# EUROPEAN PATENT SPECIFICATION

(11) **EP 1 470 226 B1**
(45) Date of publication and mention of the grant of the patent: **01.08.2007**
(21) Application number: 02801387.8
(22) Date of filing: 11.10.2002
(51) Int. Cl.: C12N 15/11, C07K 14/47, C12N 15/67, A61K 38/16, A61K 47/48, C12N 5/10, A01K 67/027, C12N 15/82

(54) **CONTROL OF GENE EXPRESSION USING A COMPLEX OF AN OLIGONUCLEOTIDE AND A REGULATORY PEPTIDE**
KONTROLLE DER GENEXPRESSION DURCH VERWENDUNG EINES KOMPLEXES AUS EINEM OLIGONUCLEOTID UND EINEM REGULATORISCHEN PEPTID
REGULATION DE L'EXPRESSION GENIQUE AU MOYEN D'UN COMPLEXE CONSTITUE D'UN OLIGONUCLEOTIDE ET D'UN PEPTIDE REGULATEUR

(30) Priority: 11.10.2001 GB 0124391
(43) Date of publication of application: 27.10.2004
(73) Proprietor: Imperial Innovations Limited, Imperial College London SW7 2AZ (GB)
(72) Inventor: HART, Stephen Div. of Medicine, Cancer Cell Bio. Sect., R. 601b, Du Cane Rd., London W12 0NN (GB); ALI, Simak Div. of Medicine, Cancer Cell Bio. Sect., R. 601b, De Cane Rd., London W12 0NN (GB); PUFONG, Boris, Tumi Cyprotex Discovery Ltd, Macclesfield SK10 2 DR (GB); PORTER, Andrew, Christopher, George MRC Clinical, London W12 0NN (GB); BULUWELA, Laki Div. of Medicine, Cancer Cell Bio., Du Cane Rd., London W12 0N (GB); VAINIKKA, Satu, London W1J 5PT (GB); JENKINSON, John, David Cancer Research Technology Ltd, London WC2A 3NL (GB); KANDA, Patrick Activotec-SSP, Ltd., Biomedical Sc, Southampton SO16 7PX (GB)
(74) Representative: Pilkington, Stephanie Joan
(86) International application number: PCT/GB2002/004633
(87) International publication number: WO 2003/033701

(56) References cited:
- WO-A-01/02019
- WO-A-01/59094
- WO-A-99/11809
- LEBEDEVA I ET AL: "Cellular delivery of antisense oligonucleotides" EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 101-119, XP004257182 ISSN: 0939-6411
- STRUHL K: "histone acetylation and transcriptional regulatory mechanisms" GENES AND DEVELOPMENT, COLD SPRING HARBOR LABORATORY PRESS, NEW YORK, US, vol. 12, 1998, pages 599-606, XP002157338 ISSN: 0890-9369

## Description

The present invention relates to the control of gene expression and, in particular, it relates to methods of, and means for, modulating, preferably suppressing, the expression of a particular, selected gene.

The ability to selectively suppress the expression of a gene is useful in many areas of biology, for example in methods of treatment where the expression of the gene may be undesirable; in preparing models of disease where lack of expression of a particular gene is associated with the disease; in modifying the phenotype in order to produce desirable properties. Thus, the ability to selectively suppress the expression of a gene may allow the "knockout" of human genes in human cells (whether wild type or mutant) and the knockout of eukaryotic genes in studies of development and differentiation.

Present methods of attempting to suppress the expression of a particular gene fall into three main categories, namely antisense technology, ribozyme technology and targeted gene deletion brought about by homologous recombination.

Antisense techniques rely on the introduction of a nucleic acid molecule into a cell which typically is complementary to a mRNA expressed by the selected gene. The antisense molecule typically suppresses translation of the mRNA molecule and prevents the expression of the polypeptide encoded by the gene, whilst the antisense molecule remains bound to the mRNA molecule. Modifications of the antisense technique may prevent the transcription of the selected gene by the antisense molecule (triplex forming oligonucleotide; TFO) binding to the gene's DNA to form a triple helix. In this method, the presence of the third strand blocks DNA transcription whilst it remains bound.

Chemical modifying groups, for example psoralen cross-linking groups, have been included in TFOs, but these can lead to irreversible DNA damage and mutation. Controlling such chemical modifying groups in cells is also difficult. They may also have disadvantages in relation to cellular delivery of the molecules.

Ribozyme techniques rely on the introduction of a nucleic acid molecule into a cell which expresses a RNA molecule which binds to, and catalyses the selective cleavage of, a target RNA molecule. The target RNA molecule is typically a mRNA molecule, but it may be, for example, a retroviral RNA molecule.

Antisense- and ribozyme-based techniques have proven difficult to implement and they show varying degrees of success in target gene suppression or inactivation. Furthermore, these two techniques require persistent expression or administration of the gene-inactivating agent

Linkage of a TFO to a VP16 viral activation domain (Kusnetsova et al (1999) Nucleic Acids Res 20, 3995-4000) has been used to broaden the application of TFOs to include gene activation (as opposed to previous uses in gene suppression or inactivation).

Targeted gene deletion by homologous recombination requires two gene-inactivating events (one for each allele) and is not easily applicable to primary cells, particularly for example primary human mammary cells which can only be maintained in culture for a few passages. Targeted gene deletion has remained difficult to perform in plants. The *cre-lox* mediated site-specific integration has been the method of choice although the efficiency of specific integrative events is low (Alberts et al (1995) Plant J. 7, 649-659; Vergunst & Hooykass (1998) Plant Mol. Biol. 38, 393-406; Vergunst et al (1998) Nucl. Acids Res. 26, 2729-2734).

These major shortcomings in existing technology have led us to seek an alternative strategy.

WO 99/11808 describes fusion proteins or protein/nucleic acid complexes comprising antennapedia protein.

WO 01/02019 describes fusions of two polypeptides in which one polypeptide is capable of specifically binding to a selected nucleic acid sequence and the other polypeptide inactivates chromatin.

Lebedeva et al (2000) Journal of Pharmaceutics and Biopharmaceutics 50, 101-119 describes reviews methods for delivery of oligonucleotides into cells.

An aspect of the invention provides an in vitro method for suppressing the expression of a selected gene in a cell the method comprising introducing into the cell a molecule comprising (1) a nucleic acid binding portion which binds to a site at or associated with the selected gene which site is present in a genome and (2) an expression repressor portion, wherein the nucleic acid binding portion comprises an oligonucleotide or oligonucleotide mimic or analogue, and wherein the repressor portion comprises a polypeptide or peptidomimetic.

A further aspect of the invention provides an in-vitro method for modulating the expression of a selected gene in a cell the method comprising introducing into the cell a molecule comprising (1) a nucleic acid binding portion which binds to a site at or associated with the selected gene which site is present in a genome and (2) a modifying portion, wherein the nucleic acid binding portion comprises an oligonucleotide or oligonucleotide mimic or analogue, and wherein the modifying portion comprises a polypeptide or peptidomimetic which is capable of modulating covalent modification of nucleic acid or chromatin and is not an endonuclease or KRAB.

A further aspect of the invention provides a molecule comprising (1) a nucleic acid binding portion which binds to a site at or associated with a selected gene which site is present in a genome and (2) an expression repressor portion, wherein the nucleic acid binding portion comprises an oligonucleotide or oligonucleotide mimic or analogue, and wherein the repressor portion comprises a polypeptide or peptidomimetic.

A further aspect of the invention provides a molecule comprising (1) a nucleic acid binding portion which binds to a site at or associated with a selected gene which site is present in a genome and (2) a modifying portion, wherein the nucleic acid binding portion comprises an oligonucleotide or oligonucleotide mimic or analogue, and wherein the modifying portion comprises a polypeptide or peptidomimetic which is capable of modulating covalent modification of nucleic acid or chromatin and is not an endonuclease or KRAB.

It is preferred that the cell or genome is a eukaryotic cell or genome, for example a fungal animal or plant cell.

It is preferred that the repressor portion is a modifying portion. It is preferred that the repressor or modifying portion is a chromatin inactivation portion. The chromatin inactivation portion may be any polypeptide or part thereof which directly or indirectly leads to chromatin inactivation. By "directly" leading to chromatin inactivation we mean that the polypeptide or part thereof itself acts on the chromatin to inactivate it By. "indirectly" leading to chromatin inactivation we mean that the polypeptide or part thereof does not itself act on the chromatin but rather it is able to recruit or promote a cellular component to do so.

Chromatin inactivation generally results in the suppression or inactivation of gene expression. Chromatin inactivation is typically a localised event such that suppression or inactivation of gene expression is restricted to, typically, one or a few genes. Thus, the chromatin inactivation portion is any suitable polypeptide which, when part of the polypeptide of the invention and when targeted to a selected gene by the nucleic acid binding portion, locally inactivates the chromatin associated with the selected gene so that expression of the gene is inactivated or suppressed. Histone deacetylation is associated with chromatin inactivation and so it is particularly preferred if the chromatin inactivation portion facilitates histone deacetylation. Targeted deacetylation of histones associated with a given gene leads to gene inactivation in an, essentially, irreversible manner. By "suppression" or "inactivation" of gene expression we mean that in the presence of the polypeptide of the invention the expression of the selected, targeted gene is 1.2-fold, 1.4-fold, 1.6-fold, two-fold, three-fold, five-fold, ten-fold, twenty-fold, 50-fold, 100-fold, or 1000-fold lower than in the absence of the polypeptide of the invention under equivalent conditions. Gene expression can be measured using any suitable method including using reverse transcriptase-polymerase chain reaction (RT-PCR), RNA hybridisation, RNAse protection assays, nuclear run-off assays and alteration of chromatin as judged by DNAse 1 hypersensitivity.

In animal and plant cells histone deacetylation is brought about by the so-called histone deacetylase complex (HDAC) which contains, in addition to one or more histone deacetylase enzymes, ancillary proteins which are involved in the formation and function of the complex. In addition, there are other protein components which although they may not be part of **HDAC they bind to or otherwise interact with HDAC and help facilitate histone deacetylation.**

Deacetylation and acetylation of histones is a well-known phenomenon which is reviewed in the following: Chen & Li (1998) Crit. Rev. Eukaryotic Gene Expression 8, 169-190; Workman & Kingston (1998) Ann. Rev. Biochem. 67, 545-579; Perlmann & Vennstrom (1995) Nature 377, 387- ; Wolfe (1997) Nature 387, 16-17; Grunstein (1997) Nature 389, 349-352; Pazin & Kadonaga (1997) Cell 89, 325-328; DePinho (1998) Nature 391, 533-536; Bestor (1998) Nature 393, 311-312; and Grunstein (1998) Cell 93, 325-328.

The polypeptide composition of the HDAC complex is currently under investigation. Polypeptides which may form part of, or are associated with, certain HDAC complexes include histone deacetylase 1 (HDAC1) Taunton et al (1996) Nature 272, 408-441); histone deacetylase 2 (HDAC2) (Yang et al (1996) Proc. Natl. Acad. Sci. USA 93, 12845-12850); histone deacetylase 3 (HDAC3) (Dangond et al (1998) Biochem. Biophys. Res. Comm. 242, 648-652); N-CoR (Horlein et al (1995) Nature 377, 397-404); SMRT (Chen & Evans (1995) Nature 377, 454-457); SAP30 (Zhang et al (1998) Molecular Cell 1, 1021-1031). Sin3 (Ayer et al (1995) Cell 80, 767-776; Scbreiber-Agus et al (1995) Cell 80, 777-786) SAP18 (Zhang et al (1997) Cell 89, 357-364); and RbAp48 (Qian et al (1993) Nature 364, 648-652). All of these papers are incorporated herein by reference. It is believed that there may be further components of the HDAC complex or which interact with the HDAC complex which are, as yet, undiscovered. It is envisaged that these too will be useful in the practice of the invention.

PLZF has been shown to interact with N-CoR and SMRT, which in turn recruit a HDAC complex. PLZF will also directly interact with HDAC (Lin et al (1998) Nature 391, 811-814; Grignani et al (1998) Nature 391, 815-818; David et al (1998) Oncogene 16, 2549-2556).

Mad1 is a member of the Mad family and has an ability to act as a transcriptional repressor. It has been shown that Mad1 is able to interact with Sin3, which in turn interacts with class I histone deacetylases (HDAC1 and HDAC2). Mad/Sin3 functions as a large protein scaffold capable of multiple protein - protein interactions (Hassig et al (1997) Cell 89, 341-347; Laherty et al (1997) Cell 89, 349-356; Zhang et al (1997) Cell 89, 357-364)).

Complexes formed which contain any of N-CoR, SMRT, Sin3, SAP18, SAP30 and histone deacetylase are described in Heinzel et al (1997) Nature 387, 43-48; Alland et al (1997) Nature 387, 49-55; Hassig et al (1997) Cell 89, 341-347; Laherty et al (1997) Cell 89, 349-356; Zhang et al (1997) Cell 89, 357-364; Kadosh & Stuhl (1997) Cell 89, 365-371; Nagy et al (1997) Cell 89, 373-380; and Laherty et al (1998) Molecular Cell 2, 33-42. All of these papers are incorporated herein by reference.

Thus, it is particularly preferred if the component of a HDAC complex or the polypeptide which binds to or facilitates recruitment of a HDAC complex is any one of MAD1, E7, PLZF, SMRT, Sin3, SAP18, SAP30 or N-CoR, or HDACs including HDAC1, HDAC2 or HDAC3, or NuRD, MAD2, MAD3, MAD4 or Rb. It will be appreciated that it may not be necessary for all of the polypeptides to be present so long as a functional portion thereof is present. For example, with respect to histone deacetylase enzymes (for example, HDAC1, HDAC2 or HDAC3) the functional portion may be a portion that retains histone deacetylase activity or it may be a portion which contains a binding site for other components of a HDAC complex or a portion which otherwise recruits the HDAC complex and promotes histone deacetylation. Similarly, with respect to other components of the HDAC complex or polypeptides which bind to the HDAC complex the functional portion may be a portion which contains a binding site for other components of the HDAC complex. To date six mammalian HDAC genes have been described (Grozinger et al (1999) Proc. Natl. Acad. Sci. USA 96, 4868-4873), it is believed that any one or more of these may be useful in the practise of the present invention.

For the avoidance of doubt, VP16 or KRAB are not included within the meaning of the term "modifying portion" or "chromatin inactivation portion". VP16 is a transcriptional activator whose mode of action is not considered to involve covalent modification of DNA or chromatin. KRAB is a transcriptional repressor whose mode of action is considered to involve mechanisms other than chromatin inactivation. Although not preferred, any fragment of KRAB that, when part of the molecule/polypeptide as defined above and when targeted to a selected gene by the nucleic acid binding portion, locally inactivates the chromatin associated with the selected gene so that expression of the gene is inactivated or suppressed, is included within the term "chromatin inactivation portion". For example, any fragment of KRAB that is capable of binding to or facilitating recruitment of a HDAC complex is included within the term "chromatin inactivation portion". However, any such fragments are not preferred.

It is believed that binding motifs are present within the components of the HDAC complex or within polypeptides which bind the HDAC complex and these motifs may be sufficient to act as chromatin inactivation portions in the polypeptide of the invention since they may facilitate histone deacetylation by recruiting a HDAC complex.

Furthermore, it will be appreciated that variants of a component of the HDAC complex or variants of a polypeptide which binds to the HDAC complex may be used. Suitable variants include not only functional portions as described above, but also variants in which amino acid residues have been deleted or replaced or inserted provided that the variant is still able to facilitate histone deacetylation. Thus, suitable variants include variants of histone deacetylase in which the amino acid sequence has been modified compared to wild-type but which retain their ability to deacetylate histones. Similarly, suitable variants include variants of, for example, Sin3 or PLZF in which the amino acid sequence has been modified compared to wild-type but which retain their ability to interact with or in the HDAC complex. Similarly, variants of other proteins interacting with components of the HDAC complex and other transcription factors that can bring about gene inactivation through HDAC activity may be used.

All or parts of the Rb, MAD and MeCpG2 proteins may interact with the HDAC complex.

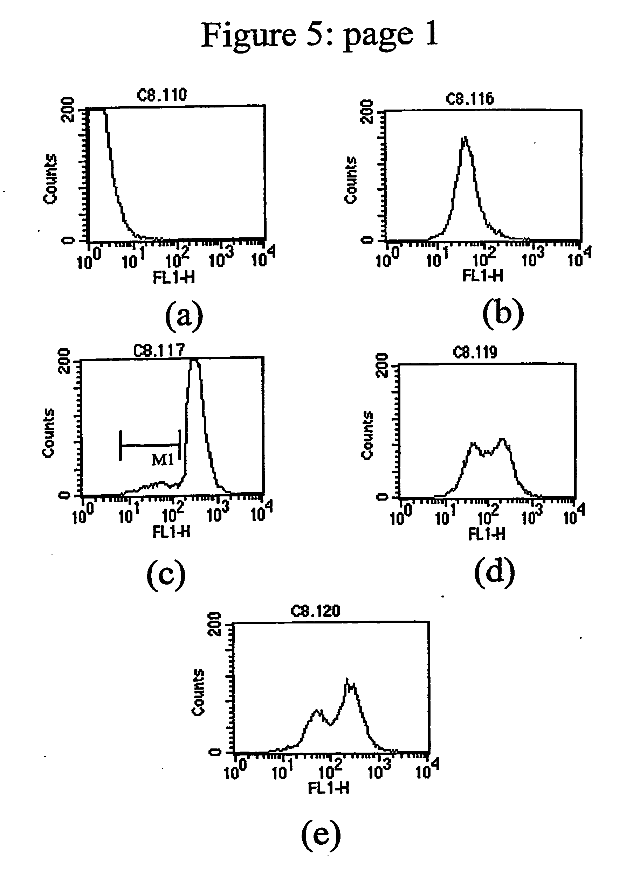

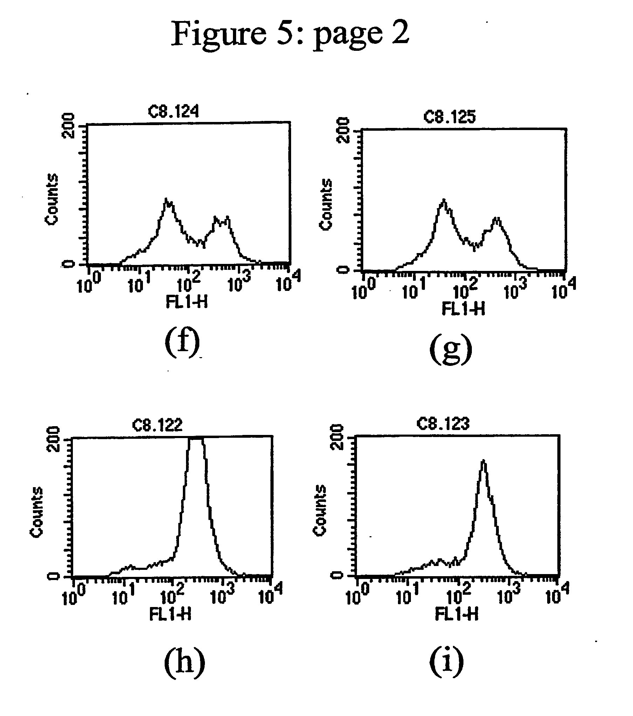

While most work has been done on HDAC complexes and polypeptides **involved in recruiting HDAC complexes in mammalian systems, the** fundamental nature of the system is such that functionally equivalent polypeptides are expected to be found in other eukaryotic cells, in particular in other animal cells and in plant cells. For example, Figure 5 shows that polypeptides very closely related to human HDAC1 are present in arabidopsis and in yeast A plant HDAC complex has been isolated from maize (Lussen et al (1997) Science 277, 88-91) and a comparative study of histone deacetylases from plant, fungal and vertebrate cells has been undertaken (Lechner et al (1996) Biochim. Biophys. Acta 1296, 181-188). Histone deacetylase inhibitors have been shown to derepress silent rRNA genes in Brassica (Chen & Pickard (1997) Genes Dev. 11, 2124-2136) and a naturally occurring host selective toxin (HC toxin) from *Cochliobolus carbonum* inhibits plant, fungal and mammalian histone deacetylases (Brosch et al (1995) Plant Cell 7, 1941-1950).

It is not necessary that the chromatin inactivation portion is from the same cell type or species as the cell into which the polypeptide (or polynucleotide encoding the polypeptide) is introduced but it is desirable if it is since such a chromatin inactivation portion may be able to inactivate chromatin more effectively in that cell.

It is particularly preferred if the chromatin inactivation portion of the polypeptide is PLZF, E7, MAD1, Rb or SAP18, or a portion of PLZF or E7 or MAD1 or Rb or SAP18 that can facilitate histone deacetylation, or a polypeptide, or portion of a polypeptide, known to cause gene activation via histone deacetylation. For example, the portion of PLZF in PLZF-RARα which is involved in APL is believed to interact with N-CoR and SMRT.

Preferred chromatin inactivation portions are described in the Examples, and include a polypeptide/polypeptide mimic or analogue derivable from SAP18 with the amino acid sequence XXXMAVESRVTQEEIKKEPEKPIDREKTCPLLLRVF (where XXX is, for example, a AAA or DDD linker) and a polypeptide derivable from MAD 1 with the amino acid sequence XXXMNIQMLLEAADYLERREREAEHGYASMLP (where XXX is, for example, a AAA or DDD linker).

It is also particularly preferred if the chromatin inactivation portion is a polypeptide with histone deacetylase enzyme activity such as contained in HDAC1, HDAC2 or HDAC3.

Alternatively, the modifying portion may be a portion that is capable of modulating covalent modification, for example methylation, of nucleic acid, preferably DNA. Thus, the modifying portion may be or comprise a DNA modifying enzyme, or may be capable of recruiting such an enzyme. The modulation preferably has the effect of suppressing the selected gene.

It is preferred that the modifying portion does not change the sequence of the nucleic acid. It is preferred that the modifying portion does not cleave the nucleic acid backbone. The modifying portion is preferably not a recombinase or a restriction endonuclease.

For example, the modifying portion may comprise (or be capable of recruiting) all or a portion of a methyl transferase or a component of a methyltransferase complex, for example as discussed in Okano M, Xie S, Li E. (1998) Cloning and characterization of a family of novel mammalian DNA (cytosine-5) methyltransferases. Nat Genet 19:219-220; Adrian P. Bird and Alan P. Wolffe (1999) Methylation-Induced Repression: Belts, Braces, and Chromatin. Cell 99,451-454.

It is preferred that the repressor or modifying portion is not an endonuclease or other molecule that produces a persistent break in the DNA strand.

It is preferred that a polypeptide/polypeptide mimic or analogue portion of the molecule (for example the modifying portion) has a molecular mass of less than 11 kDa, preferably less than 8 kDa, still more preferably less than 6 kDa. For example, it is preferred that the polypeptide/polypeptide mimic or analogue portion has less than 100, still more preferably less than 90, 80, 70, 60, 50, 45, 40, 35, 30, 25 or 20 amino acids (or mimics or analogues thereof), most preferably between about 60 and 25 amino acids (or mimics or analogues thereof).

It is particularly preferred that the modifying portion consists of peptides derivable from SAP18 or MAD 1 or Rb and appropriate linkers, for example the peptides derivable from SAP18 or MAD1 and linkers as described above and in Example 1.

The molecule may further comprise a portion which facilitates cellular entry and/or nuclear localisation (locating portion). This portion may also be a polypeptide or polypeptide mimic/analogue. For example, the locating portion may comprise or consist of a peptide with membranotropic activity as discussed, for example, in Soukchareun et al (1998) Bioconjugate Chem 9, 466-475 and references cited therein, for example Soukchareun et al (1995) Bioconjugate Chem 6, 43-53 (viral fusion peptides) or Eritja et al (1991) Tetrahedron 47, 4113-4120 (nuclear transport signal sequences). It may be a nuclear localisation signal peptide or endosomal lytic peptide (which may facilitate release of the molecule from the endosomal compartment) mentioned in WO 99/13719. It is preferred that this portion is of less than 3 kDa, preferably of less than 2.5 kDa. It is preferred that the total polypeptide/mimic/analogue content of the molecule is less than 11 kDa. Typically, a localisation portion may have between about 7 and 16 amino acids.

Further examples of localisation portions include modified Antennapedia homeodomain based Penetratins (for example RQIKIWFQNRRMKWKK), or TAT (for example C(Acm)GRKKRRQRRRPPQC, where C(Acm) is a Cys-acetamidomethyl) or VP22 based molecules (Prochiantz (2000) Curr Opin Cell Biol 9, 420-429).) or basic HIV TAT internalisation peptide.

The molecules of the invention may be useful in methods and uses provided by aspects of the invention, for example as discussed in more detail below. In particular, the polypeptides of the invention may be useful in a method of the first or second aspect of the invention.

It is preferred if the molecules of the invention are hybrid molecules which do not occur in nature. For example, it is preferred if the nucleic acid binding portion and the modifying portion are not derivable from a naturally occurring complex or molecule. The molecules (if any) from which the nucleic acid binding portion and the chromatin inactivation portion are derived may be from the same species (for example, as is described in more detail below, the nucleic acid binding portion may be an oligonucleotide having a sequence found in human nucleic acid and the chromatin inactivation portion may be a portion of human PLZF) or they may be from different species (for example an oligonucleotide having a sequence not found in human nucleic acid, for example capable of binding to a bacterial DNA sequence, may be fused to a portion of human PLZF).

Thus, in a particular preferred embodiment the molecule of the invention is one which is produced by chemical synthesis methods wherein the nucleic acid binding portion and the modifying or chromatin inactivation portion are selected as is described in more detail below.

Synthesis and joining techniques are discussed in WO 01/14737 and references therein (incorporated herein by reference). The methods of WO 01/147373 are preferred. Alternatively, techniques described in Kusnetsova et al (1999) Nucleic Acids Res 27, 3995-4000 may also be used.

The site present in a eukaryotic genome is a site which is at or associated with a selected gene or genes whose expression it is desirable to modulate, preferably suppress or inactivate. It is preferred if the site is a site which is naturally present in a eukaryotic genome. However, as is discussed in more detail below, the site may be one which has been engineered into the genome, or it may be a site associated with an inserted viral sequence. The site engineered into the genome to be in the vicinity of the gene whose expression is to be suppressed may be a site from the same species (but present elsewhere in the genome) or it may be a site present in a different species. By "genome" we include not only chromosomal DNA but other DNA present in the eukaryotic cell, such as DNA which has been introduced into the cell, for example plasmid or viral DNA. It is preferred if the nucleic acid binding portion can bind to chromosomal DNA or, as is described in more detail below, to RNA transcribed from chromosomal DNA.

In an embodiment, it is preferred that the gene is an endogenous gene. The term "endogenous gene" refers to a gene that is native to the cell ie which is not heterologous to the cell and is in its natural genomic context. In this context the site present in a eukaryotic genome is a site which is at or associated with the selected endogenous gene or genes whose expression it is desirable to suppress or inactivate. The site is a site which is naturally present in a eukaryotic genome and is in its natural genomic context.

It may be desirable for the site to have particular sequence characteristics that promote binding to an oligonucleotide to form a triple helix, as known to those skilled in the art. However, it is considered in relation to the present invention that such sequence characteristics may be less important than for oligonucleotides in the absence of a polypeptide portion, because the suppressing or modulating effect of the molecule of the invention may persist even when the molecule is no longer bound to the target site; thus the affinity of binding may be less critical. The sequence of the oligonucleotide is still important so that specific recognition is obtained; however the bonds that are formed between oligo and target sequence may not need to be as strong when the polypeptide/peptidomimetic portion is present.

Positioning of the oligonucleotide binding site relative to the gene whose transcription is to be suppressed or modulated may also be less critical than for oligonucleotides, for example TFOs, without a modifying portion as the modulating or suppressing effect of the molecule of the invention (for example when the modifying domain is or is capable of recruiting a methyltransferase or histone deacetylase) may extend to either side of the oligonucleotide binding site. The nucleic acid binding portion may bind to the gene promoter, but may alternatively bind to another sequence within or in proximity to the gene of interest.

WO 90/06934/EP 0 375 408 and WO 91/06626 discuss sequence requirements for TFOs. Two motifs for the formation of a triple helix are termed the "CT"' motif and the "GT" motif. The first of these involves the use of a polypyrimidine oligonucleotide as the TFO. For every GC base pair, a C is present in the TFO and for every AT base pair, a T (or xanthine or inosine or a halogenated derivative) is present in the TFO. The TFO is considered to be oriented in a parallel direction to the purine-rich strand of the duplex. Alternatively, using the "GT" motif, a G (or halogenated derivative) is present for every GC base pair and a T (or xanthine or inosine or a halogenated derivative) for each AT base pair, and the TFO is considered to be oriented in an anti-parallel direction to the purine-rich strand of the duplex. The target sequence should have at least about 65% purine bases or at least about 65% pyrimidine bases. EP 0 266 099 also discusses how suitable target sequences may be selected.

WO94/17086 discusses oligonucleotides that are intended to bind to DNA sequences that are considered to be capable of adopting a single-stranded conformation. Such sequences may be purine-rich and have substantial mirror symmetry. The oligonucleotides may be substantially complementary to the purine strand, or may have a circular or stem-loop functioning structure that may form both Watson-Crick and Hoogensteen bonds with the single-stranded target DNA.

WO96/35706 describes oligonucleotides with structures and sequence characteristics that are considered to promote specific and stable complex formation with target nucleic acid (pyrimidine single-stranded nucleic acids) and which may have greater stability due to formation of a parallel-stranded hairpin structure in the absence of target nucleic acid.

Debin et al (1999) Nucl Acids Res 27(13), 2699-2707 comments on factors affecting the stability of G,A triple helices and the consequences for TFO design. Xodo et al (2001) Eur J Biochem 268, 656-664 also investigates factors affecting TFO binding to target sites, for example binding of short oligonucleotides to neighbouring sites.

Blume et al (1999) Nucl Acids Res 27, 695-702 investigates the involvement of a divalent cation in triple helix formation and how formation may be positively or negatively modulated. Faria et al (2001) J Mol Biol 306, 15-24 describes an assay for evaluating TFOs in cells and results with various oligonucleotides. Cheng et al (2000) Biotech and Bioeng 70, 467-472 presents the results of mathematical modelling of TFO bindings and the consequences for choosing binding sites and TFO sequences.

Demidov & Frank-Kamenetskii review binding of peptide nucleic acids (PNAs), particularly cationic pyrimidine PNAs (cpyPNAs) to duplex DNA.

Rules for designing potential TFOs are reviewed in Vasquez & Wilson (1998) Trends Biochem Sci 1, 4-9. Three types of TFOs are indicated to be effective: pyrimidine rich (CT); purine rich (GA) and mixed (GT or GAT). CT TFOs bind in a parallel motif, in which the third strand has the same 5' to 3' orientation as the purine strand of the duplex. GA TFOs bind in an antiparallel motif. Mixed TFOs may bind in either manner, depending on the target sequence. Other properties also differ between the types of TFOs; for example CT TFOs are pH dependent. Each type of TFO may be suitable in relation to the present invention.

It is preferred that the oligonucleotide or mimic portion is about 10 to 80, preferably 15 to 40 bases long, still more preferably about 20 to 40 bases long. Oligonucleotides of less than 20 bases may display weaker and/or less specific binding but may nevertheless be useful in the practice of the invention, for example because only transient binding is required, as noted above.

By "DNA" or "oligo(deoxy)nucleotide" we mean a molecule with a sugar-linkage-sugar backbone wherein the sugar residue comprises a 2'-deoxyribose (and therefore includes a DNA chain terminated with a nucleoside comprising a 2',3' dideoxyribose moiety) and wherein, attached to the sugar residue at the 1 position is a base such as adenine (A), cytosine (C), guanine (G), thymidine (T), inosine (1), uridine (U) and the like. In normal DNA the linkage between sugar residues (the "sugar-sugar linkage") is a phosphate moiety which forms a diester with the said sugar residues. However, we include in the term "nucleic acid" (and more particularly in the term DNA) molecules with non-phosphate linkages.

Thus, we include a phosphorothioate linkage and a phosphoroselenoate linkage. It may be preferred that the linkages are more resistant to attack by cellular nucleases than normal DNA. Such linkages may also include methyl phosphate, phosphotriester and the α enantiomer of naturally occurring phosphodiester.

By the terms "nucleic acid" or "oligonucleotide" we also include molecules with non-natural base analogues; molecules in which the 2' and 3' positions of the pentose sugar are independently any of -H, -OH or -NH₂; and molecules in which an oxygen attached to the phosphorus atom but not in phosphodiester linkage is replaced by -SH, SeH, -BH₂, -NH₂, -PH₃, -F, -Cl, -CH₃, -OCH₃, -CN and -H.

The oligonucleotide may be a oligoribonucleotide or a oligodeoxyribonucleotide. Oligodeoxyribonucleotides are preferred as oligoribonucleotides may be more susceptible to enyzymatic attack than oligodeoxyribonucleotides.

The oligonucleotide or analogue or mimic may be a peptide nucleic acid, as known to those skilled in the art and described in, for example WO 99/13719 and references therein, and in Demidov & Frank-Kamenetskii (2001) *supra.* PNAs are nucleic acid analogs with a polyamide (peptide) backbone containing 2-aminoethyl glycine units in place of the deoxyribose-phosphate backbone of DNA. The PNA backbone is neutral (unlike the DNA backbone, which is negatively charged) and may therefore bind more stably to a charged nucleic acid molecule than would the corresponding DNA molecule.

It is preferred that the oligonucleotide or analogue or mimic is a DNA oligonucleotide.

References to an oligonucleotide include (where appropriate) reference to an oligonucleotide mimic or analogue, for example a PNA.

The oligonucleotide may comprise a linker, which may be attached to the 5' or 3' terminus of the oligonucleotide. Examples of suitable linkers are described in, for example, WO 90/06934.

It may be preferred that the nucleic acid binding portion is or comprises a peptide nucleic acid (PNA).

The nucleic acid binding portion may be any suitable binding portion as defined which binds to a site present in a eukaryote, such as a plant or animal, genome. It is particularly preferred that the nucleic acid binding portion is able to bind to a site which is at or associated with a selected gene whose expression is to be suppressed by the presence of the chromatin inactivating portion of the polypeptide of the invention. It is preferred that the nucleic acid binding portion binds selectively to the desired site. There may be one or more desired sites to which the nucleic acid binding portion may bind. For example, the polypeptide of the invention may be used to suppress the expression of a group of genes which each have a binding site for a common DNA binding portion (for example, are under the controls of a steroid hormone receptor such as the oestrogen receptor (ER)). For the avoidance of doubt, the site present in the eukaryote may be a naturally occurring site, or it may be a site which has been engineered to be there. The site need not be originally from the same or any other eukaryote. For example, it may be a bacterial or viral sequence or artificial sequence for which TFOs have previously been characterised, which has been engineered to be present in the DNA of the eukaryotic cell, for example a plant cell.

Examples may include response elements, such as ERE and IRE as described in examples here, or other characterised binding sites. It may be desirable to use such a site in a cell which does not contain an endogenous regulator of the site. Alternatively, the site may be a modified version of a naturally occuring response element, which modified version may serve as a binding sites for TFOs, but may not be regulated by a naturally occuring regulator of the naturally occuring response element. However, it is preferred if the site to which the nucleic acid binding portion binds is naturally present in the eukaryotic cell and is present in its natural position in the genome.

The nucleic acid binding portion may be a DNA binding portion or an RNA binding portion. Thus, the nucleic acid binding portion may bind to double-stranded nucleic acid (for example DNA) or to single-stranded nucleic acid (for example RNA or single-stranded DNA). In the case of the RNA binding portion, the site present in the eukaryotic genome which binds the RNA binding portion is, typically, nascent RNA being transcribed from DNA at the selected site for inactivation. The RNA may be that which is being transcribed by the gene whose expression is to be suppressed, or it may be that which is being transcribed by a gene adjacent to, or at least close to, the gene whose expression is to be suppressed. It is preferred that the RNA binding portion binds to an RNA sequence which is at or close to the 5' end of the transcript. It will be appreciated that whilst being transcribed, nascent RNA remains at or close to its site of transcription and that if the site of transcription is at or close to the gene whose expression is to be suppressed, using an RNA binding portion in the molecule of the invention facilitates the localisation of the chromatin inactivation portion to the desired site.

It may be useful if the DNA binding portion binds to a transcription factor binding site, for example so that expression of more than one gene to which the transcription factor binds may be modulated. Databases listing transcription factors and their binding sites are listed below:
http://www.embl-heidelberg.de/srs5bin/cgi-bin/wgetz?-fun+pagelibinfo+-info+TFFACTOR
http://www.embl-heidelberg.de/srs5bin/cgi-bin/wgetz?-fun+pagelibinfo+-info+TFSITE
http://www.embl-heidelberg.de/srs5bin/cgi-bin/wgetz?-fun+pagelibinfo+-info+TFCELL
http://www.embl-heidelberg.de/srs5bin/egi-bin/wgetz?-fun+pagelibinof+-info+TFCLASS
http://www.embl-heidelberg.de/srs5bin/cgi-bin/wgetz?-fun+pagelibinfo+-info+TFMATRIX
http://www.embl-heidelberg.de/srs5bin/cgi-bin/wgetz?-fun+pagelibinfo+-info+TFGENE

It may be useful if the DNA binding portion binds to a promoter region or other regulatory regions or sequences just upstream of the transcription start site. In some applications it may be preferred to target sequences within the gene in order to differentiate amongst splice variants.

As noted, oligonucleotides may be designed/engineered so as to bind to a particular, selected target DNA sequence which is at or associated with a selected gene. In one embodiment of the invention the oligonucleotide is one which has been engineered to bind to a site which is present in a mutant gene sequence within the plant or animal cell but is not present in the equivalent wild type sequence. For example, and as is discussed in more detail below, the oligonucleotide may bind selectively to a dominant negative, mutated gene, such as a mutant oncogene and, upon binding, DNA methylation or chromatin inactivation occurs and suppresses the expression of the mutant oncogene. Examples of oncogenes mutated in human cancer include RAS *(H-ras)* and *Bcl-10.*

Typically, the nucleic acid binding portion and the modifying or chromatin inactivation portion are fused. The nucleic acid binding portion and modifying or chromatin inactivation portion may be synthesised as a single molecule (total synthesis approach), for example by consecutive assembly of the peptide and then the oligonucleotide on a solid support, for example as described in Soukchareun et al (1998) supra and references cited therein, or in Basu et al (1995) Tetrahedron Lett 36, 4943. Preferably, an automated procedure is used.

Alternatively, the nucleic acid binding portion and modifying or chromatin inactivation portion are synthesised separately, using techniques well known to those skilled in the art, and then joined. Techniques suitable for the coupling of peptide nucleic acids to peptides include the use of heterobifunctional conjugation reagents such as SPDP (N-succinimidyl 3-(2-pyridyldithio)propionate) and SMCC (succinimidyl 4-(N-maleimidomethyl) cylohexene-carboxylate) and are described, for example, in WO 99/13719, particularly in Examples 12 to 15. Techniques suitable for coupling oligodeoxynucleotides to peptides include the use of N^{α}-Fmoc-cysteine(*S*-thiobutyl) derivatised oligodeoxynucleotides, as described in Soukchareun *et al* (1998) *supra.* Other techniques include the use of *N-*hydroxybenzotriazole (HOBT) ester activation of the 3' or 5' ends of oligonucleotide phosphates prior to coupling of an unprotected peptide *via* a nucleophilic group (such as an α-NH₂ group) in the peptide (see Ivanovskaya et al (1995) Nucl Nucl 6, 931-934; Ivanovskaya et al (1987) Dokl Acad Nauk SSSR 293, 477-481; Kuznetsova et al (1999) Nuc Acids Res 27, 3995-4000). Peptide-olignucleotide conjugation techniques are reviewed in, for example, Tung & Stein (2000) Bioconjugate Chem 11(5), 605-618.

Preferably, a "native ligation" technique is used, as described in WO 01/15737 and Stetsenko & Gait (2000) Organic Chem 65(16), 4900-4908.. A N-terminal thioester-functionalised peptide is ligated to a 5'-cysteinyl oligonucleotide derivative.

Suitably, the nucleic acid binding portion and the repressor, modifying or chromatin inactivation portion are joined so that both portions retain their respective activities such that, for example, the nucleic acid binding portion may bind to a site present in a plant or animal genome and, upon binding, the modifying portion is still able to modulate covalent modification of nucleic acid or chromatin, for example a chromatin inactivation portion is still able to inactivate chromatin. The two portions may be joined directly, but they may be joined by a linker peptide or oligonucleotide. Suitable linker peptides are those that typically adopt a random coil conformation, for example the polypeptide may contain alanine or proline or a mixture of alanine plus proline residues. Preferably the amino acids promote solubility; thus, the linker may contain, for example, charged or hydrophilic amino acids such as aspartic acid residues. Preferably the linker may contain or consist of aspartic acid residues. It is preferred that the amino acids are not hydrophobic amino acids, such as phenylalanine or tryptophan.
Preferably, the linker contains between 10 and 100 amino acid residues, more preferably between 10 and 50 and still more preferably between 10 and 20. A shorter linker, for example of between 3 and 9 amino acids, may also be useful. In any event, whether or not there is a linker between the portions of the molecule the molecule is able to bind its target nucleic acid and is able to repress expression or modulate covalent modification of nucleic acid or chromatin, for example inactivate chromatin thereby selectively suppressing or inactivating gene expression.

Polynucleotides which encode suitable repressor, modifying or chromatin inactivation portions are known in the art or can readily be designed from known sequences and made. Polynucleotide sequences encoding various suitable chromatin inactivation portions are given above in the references which refer to the polypeptides or are available from GenBank or EMBL or dbEST. A reference for PLZF is Chen et al (1993) EMBO J. 12, 1161-1167. A reference for E7 is Tommasino et al (1995) Bioessays 17, 509-518. References for SAP18, MAD1 and Rb are respectively Zhang et al (1997) Cell 89, 357-364; Ayer et al (1993) Cell 72, 211-222 and Weinberg (1995) Cell 81, 323-330.

Polynucleotides which encode suitable linker peptides can readily be designed from linker peptide sequences and made.

Thus, polynucleotides which encode the repressor or modifying portions of the molecules of the invention can readily be constructed using well known genetic engineering techniques. The repressor or modifying portions may therefore be synthesised by expression, using techniques of molecular biology well known to those skilled in the art. However, it may be preferred to synthesise the polypeptide/analogue/mimic portion(s) of the molecules of the invention by techniques of organic chemistry, as known to those skilled in the art and discussed herein.

The present invention also relates to a host cell transformed with a molecule of the present invention. The host cell can be either prokaryotic or eukaryotic. Bacterial cells are preferred prokaryotic host cells and typically are a strain of *E. coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include plant, yeast, insect and mammalian cells, preferably vertebrate cells such as those from a mouse, rat, monkey or human fibroblastic and kidney cell lines. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650; 293 cells which are human embryonic kidney cells, and HT1080 human fibrosarcoma cells.

Protoplasts for transformation are typically generated as required by methods known in the art. Plant cell lines are not generally available. However, one cell line which is commonly used is the Bright Yellow 2 cell line from tobacco (BY2; Mu et al (1997) Plant Mol. Biol. 34, 357-362).

Transformation of appropriate cell hosts with a molecule of the present invention is accomplished by well known methods that typically depend on the type of molecule used. With regard to transformation of prokaryotic host cells, see, for example, Cohen et al (1972) Proc. Natl. Acad. Sci. USA 69, 2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA. With regard to plant cells and whole plants the following plant transformation approaches (J. Draper and R. Scott in D. Grierson (ed.), "Plant Genetic Engineering", Blackie, Glasgow and London, 1991, vol. 1, pp 38-81) may be used:
i) DNA-mediated gene transfer, by polyethylene glycol-stimulated DNA uptake into protoplasts, by electroporation, or by microinjection of protoplasts or plant cells (J. Draper, R. Scott, A. Kumar and G. Dury, ibid., pp 161-198). Direct gene transfer into protoplasts is also described in Neuhaus & Spangenberg (1990) Physiol. Plant 79, 213-217; Gad et al (1990) Physiol. Plant 79, 177-183; and Mathur & Koncz (1998) Method Mol. Biol. 82, 267-276;
ii) transformation using particle bombardment (D. McCabe and P. Christou, Plant Cell Tiss. Org. Cult., 3, 227-236 (1993); P. Christou, Plant J., 3, 275-281 (1992)).

Preferred techniques include electroporation, microinjection and liposome formulation.

Some species are amenable to direct transformation, avoiding a requirement for tissue or cell culture (Bechtold et al (1993) Life Sciences, C.R. Acad. Sci. Paris 316, 1194-1199).

In all approaches a suitable selection marker, such as kanamycin- or herbicide-resistance, is preferred or alternatively a screenable marker ("reporter") gene, such as β-glucuronidase or luciferase (see J. Draper and R. Scott in D. Grierson (ed.), "Plant Genetic Engineering", Blackie, Glasgow and London, 1991, vol. 1 pp 38-81).

Electroporation is also useful for transforming and/or transfecting cells and is well known in the art for transforming yeast cell, bacterial cells, insect cells, vertebrate cells and some plant cells (eg barley cells, see Lazzeri (1995) Methods Mol. Biol. 49, 95-106).

For example, many bacterial species may be transformed by the methods described in Luchansky et al (1988) Mol. Microbiol. 2, 637-646 incorporated herein by reference. The greatest number of transformants is consistently recovered following electroporation of the DNA-cell mixture suspended in 2.5X PEB using 6250V per cm at 25µFD.

Methods for transformation of yeast by electroporation are disclosed in Becker & Guarente (1990) Methods Enzymol. 194, 182.

Successfully transformed cells, ie cells that contain a molecule of the present invention, can be identified by well known techniques. For example, labelled oligos and/or GFP markers may be used.

Thus, in addition to the transformed host cells themselves, the present invention also contemplates a culture of those cells, preferably a monoclonal (clonally homogeneous) culture, or a culture derived from a monoclonal culture, in a nutrient medium.

In relation to plants, it is envisaged that the invention includes single cell derived cell suspension cultures, isolated protoplasts or stable transformed plants.

Although the molecules of the invention may be introduced into any suitable host cell, it will be appreciated that they are primarily designed to be effective in appropriate animal or plant cells, particularly those that have one or more sites within their DNA to which the molecule of the invention may bind.

Thus, the animal or plant cells which contain a molecule of the invention whose presence suppresses the expression of a particular gene, or the animals or plants containing these cells, may be considered to have the gene "knocked out" in the sense that it can no longer be expressed. The chromatin inactivation by histone deacetylation may be essentially irreversible without further intervention. Repression by histone deacetylation may be reversed by using an inhibitor of histone deacetylase, for example Trichostatin A (TSA), Trapoxin or sodium butyrate (NaB), as known to those skilled in the art. Similarly, methylation may be essentially irreversible without further intervention, for example administration of methylation inhibitors/reversers, which are known in the art and include the compound azacytidine. Other methylation inhibitors include 5 deoxyazacytidine, or, for example, antisense oligos (or gene expression suppressors as described herein) directed to a DNA methyltransferase.

It will be readily appreciated that introduction of a molecule of the invention into an animal or plant cell will allow targeting of the molecule to an appropriate binding site within the nucleic acid, for example DNA (and which is bound by the DNA-binding portion of the polypeptide) and allow for suppression or inactivation or other modulation of gene expression, for example by allowing the chromatin at or associated with the target binding site to be inactivated. Typically, the molecule of the invention is selected so that it targets a selected gene. Thus, suitably, the targeted gene has a site which is bound by the DNA binding portion of the molecule associated with it The site which is so bound may be within the gene itself, for example within an intron or within an exon of the gene; or it may be in a region 5' of the transcribed portion of the gene, for example within or adjacent to a promoter or enhancer region; or it may be in a region 3' of the transcribed portion of the gene.

Genes regulated by oestrogen receptor (ER) include the progesterone receptor (PR) gene and the PS2 (trefoil related protein) gene. Thus, the method of the invention may be used to inactivate the PR gene or the PS2 gene when the DNA binding portion of the compound of the invention is able to bind to the ER binding site. Anti-oestrogen therapy is used in the treatment of breast cancer. The full repertoire of oestrogen regulated genes involved in breast cancer is presently unknown. It is generally considered that anti-oestrogen therapy results in the altered expression of key oestrogen regulated genes involved in breast cancer cell growth and transformation. The methods of the invention described below may provide an alternative, potentially more effective, way of regulating the expression (particularly inhibiting) of oestrogen-responsive genes. It may be that for certain DNA binding portions, in a given plant or animal cell there is only one target site and the expression of only one gene is suppressed or modulated by the repressor, modifying or chromatin inactivation portion. However, there may be more than one target site and introduction of a molecule of the invention may lead to suppression of expression of a number of genes.

The ability to suppress the expression of a selected gene is useful in many areas of biology.

Typically, when the gene whose expression is suppressed is in an animal cell, the animal cell is a cell within an animal and the method of the invention is used to suppress the expression of a selected gene in an animal (which may be a human or a non-human animal). Examples of particular uses in animal cells include allele-specific inactivation of oncogenic proteins such as mutant *Ras* and mutant *Bcl-10;* inhibition of oestrogen receptor regulated gene expression in breast cancer, inhibition of androgen receptor, inhibition of genes of interest for developmental studies; inhibition of genes for developing transgenic models of human diseases, for example cancer, elucidation of biochemical pathways, for example signalling pathways; drug target validation studies/cell models for diseases; and inhibition of genes involved in tissue modelling, as found in cancer and wound healing.

Also typically, the plant cell is a cell within a plant and the method of the invention is used to suppress the expression of a selected gene in a plant.

In one embodiment, the method of the invention is used to suppress the expression of socially or environmentally unacceptable or undesirable genes in commercially engineered transgenic plants. Such genes may include, for example, antibiotic or herbicide selectable marker genes. In this embodiment, the gene in the transgenic plant is targeted for silencing.

In a further embodiment of the invention novel plant architecture or floral morphology may be achieved by targeting some known homeotic genes involved in these developmental pathways.

Suitably, the method of the invention is used to suppress or inactivate the expression of a gene whose expression it is desirable to suppress or inactivate. Such genes include oncogenes, viral genes including genes present in proviral genomes and so the method in relation to animals may constitute a method of medical treatment Oncogenes may be overexpressed in certain cancers and it may be desirable to suppress their expression. Some oncogenes are oncogenic by virtue of having an activating mutation. Using the method of the invention the selective suppression of expression of a mutant oncogene may be achieved using a DNA binding portion that selectively binds to the mutant oncogene sequence and wherein the repressor or modifying portion, for example chromatin inactivation portion suppresses expression of the mutant oncogene, for example by inactivating the chromatin in which the oncogene resides or with which it is associated. Suppression of oncogene overexpression or of mutant (especially activated) oncogene expression is generally desirable in treating cancers in which the oncogenes play a role. Mutant oncogenes which may be targeted by the method of the invention include *Ras* and *Bcl-10.* These may be targeted by DNA binding portions capable of recognising the mutated genes in a sequence specific manner.

The expression of viral genes in an animal or plant cell is generally undesirable since this expression is often associated with pathogenesis. The nucleic acid of certain viruses may be formed into chromatin and the expression of such viral genes may be controlled by modification of this chromatin. For example, retroviral proviruses (ie DNA copies of retroviral RNAs) are often incorporated into animal and plant genomes where they become part of the chromatin, for example, integrated HIV provirus and integrated human papillomavirus. *Gypsy* and *Copia-*like retrotransposons appear to be widely distributed in the plant kingdom. *Copia-*like retrotransposons, or at least their reverse transcriptase domains, appear broadly distributed in higher plants while the *Gypsy-*like elements (which share their organisation with the retroviruses but lack retroviral envelope domains) are less abundant (Suoniemi et al (1998) Plant J. 13, 699-705). Integration of viral DNA into the plant genome has been demonstrated for geminiviral DNA into the tobacco nuclear genome (Bejarano et al (1996) Proc. Natl. Acad. Sci. USA 93, 759-764.). Potential retroviruses have also recently been described in plants (Wright & Voytus (1998) Genetics 149, 703-715). Using the method of the invention the selective suppression of expression of a viral gene may be achieved. The oligonucleotide/mimic/analogue nucleic acid binding domain may be used to target a repressor or modifying, for example chromatin inactivation, portion and lead to proviral genome inactivation by binding to nascent genomic RNA transcripts, achieving (for example) histone deacetylation by proximity.

Certain genetic diseases are caused by dominant mutations, such as achondroplasia. Suppression of expression of the mutant allele may be useful in treating these diseases. Using the method of the invention the selective suppression of expression of the mutant allele may be achieved using a DNA binding portion that selectively binds to the mutant allele sequence and wherein the (for example) chromatin inactivation portion inactivates the chromatin in which the mutant allele resides or with which it is associated so that expression of the mutant allele is suppressed.

These methods of the invention typically involve the transfer of the molecule of the invention into an animal or plant cell.

Tranfer systems useful with oligonucleotides or oligonucleotide-peptide fusions will be known to those skilled in the art and may be useful in the practice of the methods of the present invention in which the molecule of the invention is introduced into a cell either within or outwith an animal body.. For example, liposome or virus-based methods may be used. Electroporation (see, for example, Kuznetsova et al (1999) Nucl Acids Res 27(20), 3995-4000), ballistic methods, cationic lipids (for example as described in Felgner et al (1997) Hum Gene Ther 8, 511-512 or WO 99/13719) or specific ligands attached to the oligonucleotide or polypeptide portion of the molecule, or to the carrier may be used, for example as described in WO 99/13719.

Viral or nonviral transfer methods may be used. A number of viruses have been used as gene transfer vectors, including papovaviruses, eg SV40 (Madzak et al (1992) J. Gen. Virol. 73, 1533-1536), adenovirus (Berkner (1992) Curr. Top. Microbiol. Immunol. 158, 39-61; Berkner et al (1988) BioTechniques 6, 616-629; Gorziglia and Kapikian (1992) J. Virol. 66, 4407-4412; Quantin et al (1992) Proc. Natl. Acad. Sci. USA 89,2581-2584; Rosenfeld et al (1992) Cell 68, 143-155; Wilkinson et al (1992) Nucleic Acids Res. 20, 2233-2239; Stratford-Perricaudet et al (1990) Hum. Gene Ther. 1, 241-256), vaccinia virus (Moss (1992) Curr. Top. Microbiol. Immunol. 158, 25-38), adeno-associated virus (Muzyczka (1992) Curr. Top. Microbiol. Immunol. 158, 97-123; Ohi et al (1990) Gene 89, 279-282), herpes viruses including HSV and EBV (Margolskee (1992) Curr. Top. Microbiol. Immunol. 158, 67-90; Johnson et al (1992) J. Virol. 66, 2952-2965; Fink et al (1992) Hum. Gene Ther. 3, 11-19; Breakfield and Geller (1987) Mol. Neurobiol. 1, 337-371; Freese et al (1990) Biochem. Pharmacol. 40, 2189-2199), and retroviruses of avian (Brandyopadhyay and Temin (1984) Mol. Cell. Biol. 4, 749-754; Petropoulos et al (1992) J. Virol. 66,3391-3397), murine (Miller (1992) Curr. Top. Microbiol. Immunol. 158, 1-24; Miller et al (1985) Mol. Cell. Biol. 5, 431-437; Sorge et al (1984) Mol. Cell. Biol. 4, 1730-1737; Mann and Baltimore (1985) J. Virol. 54, 401-407; Miller et al (1988) J. Virol. 62, 4337-4345), and human origin (Shimada et al (1991) J. Clin. Invest. 88, 1043-1047; Helseth et al (1990) J. Virol. 64, 2416-2420; Page et al (1990) J. Virol. 64, 5370-5276; Buchschacher and Panganiban (1992) J. Virol. 66, 2731-2739). To date most human gene therapy protocols have been based on disabled murine retroviruses.

Nonviral gene transfer methods known in the art include chemical techniques such as calcium phosphate coprecipitation (Graham and van der Eb (1973) Virology 52, 456-467; Pellicer et al (1980) Science 209, 1414-1422); mechanical techniques, for example microinjection (Anderson et al (1980) Proc. Natl. Acad. Sci. USA 77, 5399-5403; Gordon *et al,* 1980; Brinster et al (1981) Cell 27, 223-231; Constantini and Lacy (1981) Nature 294, 92-94); membrane fusion-mediated transfer *via* liposomes (Felgner et al (1987) Proc. Natl. Acad. Sci. USA 84, 7413-7417; Wang and Huang (1989) Biochemistry 28, 9508-9514; Kaneda et al (1989) J. Biol. Chem. 264, 12126-12129; Stewart et al (1992) Hum. Gene Ther. 3, 267-275**;** Nabel *et al,* 1990; Lim et al (1992) Circulation 83, 2007-2011); and direct DNA uptake and receptor-mediated DNA transfer (Wolff et al (1990) Science 247, 1465-1468; Wu et al (1991) J. Biol. Chem. 266, 14338-14342; Zenke et al (1990) Proc. Natl. Acad. Sci. USA 87, 3655-3659; Wu *et al,* 1989b; Wolff et al (1991) BioTechniques 11, 474-485; Wagner *et al,* 1990; Wagner et al (1991) Proc. Natl. Acad. Sci. USA 88, 4255-4259; Cotten et al (1990) Proc. Natl. Acad. Sci. USA 87, 4033-4037; Curiel et al (1991a) Proc. Natl. Acad. Sci. USA 88, 8850-8854; Curiel et al (1991b) Hum. Gene Ther. 3, 147-154). Viral-mediated gene transfer can be combined with direct *in vivo* gene transfer using liposome delivery, allowing one to direct the viral vectors to the tumour cells and not into the surrounding nondividing cells.

Other suitable systems include the retroviral-adenoviral hybrid system described by Feng et al (1997) Nature Biotechnology 15, 866-870, or viral systems with targeting ligands such as suitable single chain Fv fragments.

In an approach which combines biological and physical gene transfer methods, plasmid DNA (or, for example, oligonucleotide/peptide fusion) of any size is combined with a polylysine-conjugated antibody specific to the adenovirus hexon protein, and the resulting complex is bound to an adenovirus vector. The trimolecular complex is then used to infect cells.

The adenovirus vector permits efficient binding, internalization, and degradation of the endosome before the coupled DNA is damaged.

Ebbinghaus et al (1996) Gene Ther 3(4), 287-297 describes methods by which TFOs may be delivered to cells using adenovinis-polylysine complexes. Pichon et al (2000) Nucl Acids Res 28(2**)**, describes methods by which the uptake, cytosolic delivery and nuclear accumulation of oligonucleotides may be improved, using histidylated oligolysines.

Liposome/DNA complexes have been shown to be capable of mediating direct *in vivo* gene transfer. While in standard liposome preparations the gene transfer process is nonspecific, localized *in vivo* uptake and expression have been reported in tumour deposits, for example, following direct *in situ* administration (Nabel (1992) Hum. Gene Ther. 3,399-410).

Gene transfer techniques which target the molecule directly to a target cell or tissue, is preferred. Receptor-mediated gene transfer, for example, is accomplished by the conjugation of DNA to a protein ligand via polylysine. Ligands are chosen on the basis of the presence of the corresponding ligand receptors on the cell surface of the target cell/tissue type. These ligand-DNA conjugates can be injected directly into the blood if desired and are directed to the target tissue where receptor binding and internalization of the DNA-protein complex occurs. To overcome the problem of intracellular destruction of DNA, coinfection with adenovirus can be included to disrupt endosome function.

Preferably, the method of suppressing or modulating the expression of a selected gene is used to suppress (or modulate) expression of a gene in a human cell; in one particularly preferred embodiment the human cell is within a human body.

However, the method of the invention may involve the modification of animal cells (including human cells) outside of the body of an animal (ie an *ex vivo* treatment of the cells) and the so modified cells may be reintroduced into the animal body.

The method of the invention may also involve the *in vitro* investigation or characterisation of modified cells, for example in identifying potential drug targets or screening candidate compounds for potentially pharmaceutically useful activities or properties.

From the foregoing, it will be appreciated that the method of the invention may be useful to suppress the activity of a plurality of selected genes. In particular, the method of the invention may be used to suppress the activity of a group of genes whose expression is controlled, at least to a large extent, by a single transcription factor. For example, the method may be used to suppress oestrogen-regulated genes.

The molecules of the invention, and the methods of the invention, may be used to analyse the role of genes in, amongst other things, floral development, cold regulation/adaptation, and plant responses to ethylene or pathogens these developmental and other processes.

A further aspect of the invention provides the use of a molecule of the invention in the manufacture of an agent for suppressing (or modulating) the expression of the selected gene in a (preferably eukaryotic) cell. It is preferred that the selected gene is an endogenous gene. Other preferences indicated above in relation to earlier aspects of the invention also apply.

It will be appreciated that it is particularly preferred if the molecule is used in the preparation of a medicament for suppressing (or modulating) the expression of a selected gene in an animal. For the avoidance of doubt, by "animal" we include human and non-human animals.

A further aspect of the invention provides a method of treating a patient in need of suppression (or modulation) of the expression of a selected gene, the method comprising administering to the patient an effective amount of a molecule of the invention.

It will be appreciated that suppression of the expression of a selected gene is useful where the expression or overexpression of the selected gene is undesirable and contributes to a disease state in the patient. Examples of undesirable expression of a gene include the expression of certain activated oncogenes in cancer. An increase in expression of the selected gene may be useful where the lack of or insufficient expression of the selected gene is undesirable and contributes to a disease state in the patient. For example, the insufficient expression of a tumour suppressor gene may contribute to cancer, accordingly, it may be useful to increase expression of the tumour suppressor gene.

Suppression of the expression of the ER upregulated genes is desirable in the treatment of breast cancer. Similarly, suppression of the expression of the androgen receptor (AR)-regulated genes is desirable in the treatment of prostate cancer.

Further aspects of the invention provides use of a molecule of the invention in the manufacture of a medicament for suppressing (or modulating) the expression of a selected gene in a patient in need of such suppression (or modulation).

Still further aspects of the invention provides a molecule of the invention for use in medicine. Thus, the molecule of the invention is packaged and presented for use in medicine.

Yet still further aspects of the invention provide a pharmaceutical composition comprising a molecule of the invention and a pharmaceutically acceptable carrier.

By "pharmaceutically acceptable" is included that the formulation is sterile and pyrogen free. Suitable pharmaceutical carriers are well known in the art of pharmacy.

The invention will now be described in more detail with reference to the following Figures and Examples wherein:

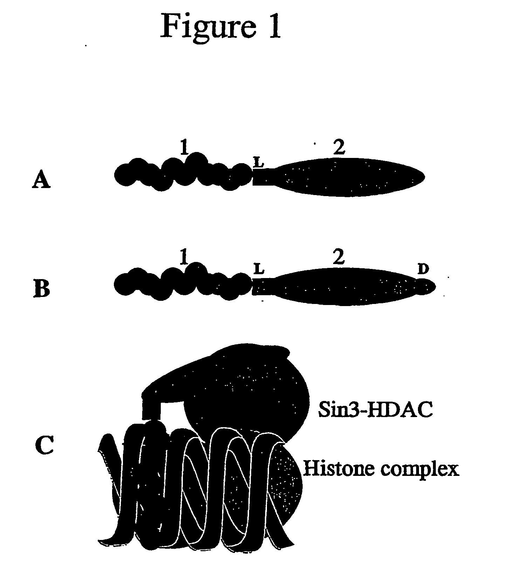

### Figure 1: Schematic representation of fusion molecules and binding to a target site

In A, "1" represents the oligonucleotide module 1 of Example 1; "2" represents the polypeptide module 2. "L" indicates a linker region. In B, D represents an additional delivery peptide. Peptides may be linked to either or both ends of the oligonucleotide; for example, the repressor/modifying polypeptide may be linked to one end and the delivery peptide to the other.

C represents the situation where the oligonucleotide portion has formed a triple helix with double-stranded DNA and the repressor peptide has recruited Sin3-HDAC complex to the site.

### Figure 2: Quantitation of androgen receptor mRNA

Columns represent the expression of mRNA as a percentage from the control value. Each column is a representative of the mean OD and standard error of the mean of four independent polymerase chain reaction. Mean optical densities were determined.

### Figure 3: Effect of ARP-L218 and R1881 on the quantity of androgen receptor mRNA.

RT-PCR analysis of androgen receptor mRNA. After ethidium bromide staining the quantity of electrophoresed amplicons was determined in arbitrary units using a Labworks image reader. The columns represent an N=1 experiment.

### Figure 4: Effect of ARP-L218 and cyproterone acetate on the quantity of androgen receptor mRNA.

RT-PCR analysis of androgen receptor mRNA. After ethidium bromide staining the quantity of electrophoresed amplicons was determined in arbitrary units using a Labworks image reader. The columns are one representative of an N=1 experiment respectively.

### Figure 5: Down regulation of interferon stimulated, genome incorporated gene.

Cells habouring EGFP under the control of the interferon stimulated response element (ISRE) of the human 6-16 gene were transiently transfected with different compounds. Cells were then treated with 300 units/ml of IFN for one day and analysed by FACS for the GFP expression.
a) Parental cell line, no treatment
b) Cells expressing the stably transfected construct (C8), no treatment
c) C8 treated with interferon (+INF)
d) C8 transfected with 500 pm TFO, +INF
e) C8 specific TFO 1000pM, +INF
f) C8, GeneICE1 500pM, +INF
g) C8, GeneICE 2 500pM, +INF
h) C8, unspecific control TFO 500pM, +INF
i) C8, unspecific control TFO 1000pM, +INF

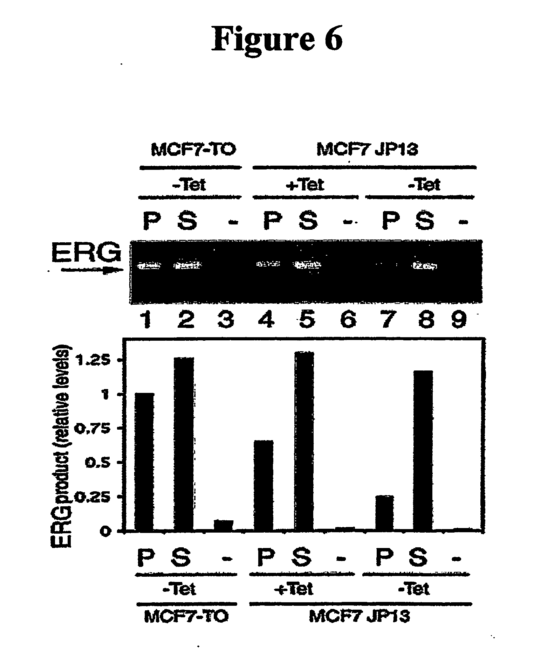

### Figure 6: Chromatin Immunopreciptation of androgen receptor DNA.

An example of the type of data that may be produced by Example 10. The data shows Chromatin Immunopreciptation of Parental MCF-7 Tet-OFF cells and PLZF-ER stably transfected cell lines. Cell lines MCF7-TO or MCF7 JP13 (with PLZF-ER, a tetracycline-inducible chromatin remodelling gene linked to a progesterone receptor DNA binding peptide) were grown to 80% confluence in phenol red free DMEM, 5% DSS, P/S/G plus selection reagents and Dox as required. Cells were treated with E2 (10⁻⁸M) or an ethanol control for 30 minutes at 37°C prior to formaldehyde cross-linking and sonication. Upper panel shows PR PCR product using DNA associated with immunoprecipitated chromatin (P) or total DNA extracted from the cells (S), using acetylated histone H4 antibody. Cells without (MCF7-TO) or with (MCF7 JP13) the PLZF-ER construct were grown in the presence (+) or absence (-) of TET. Lower panel displays PR PCR product expressed relative to the quantity of PR PCR product using DNA immunoprecipitated with an acetylated histone H4.

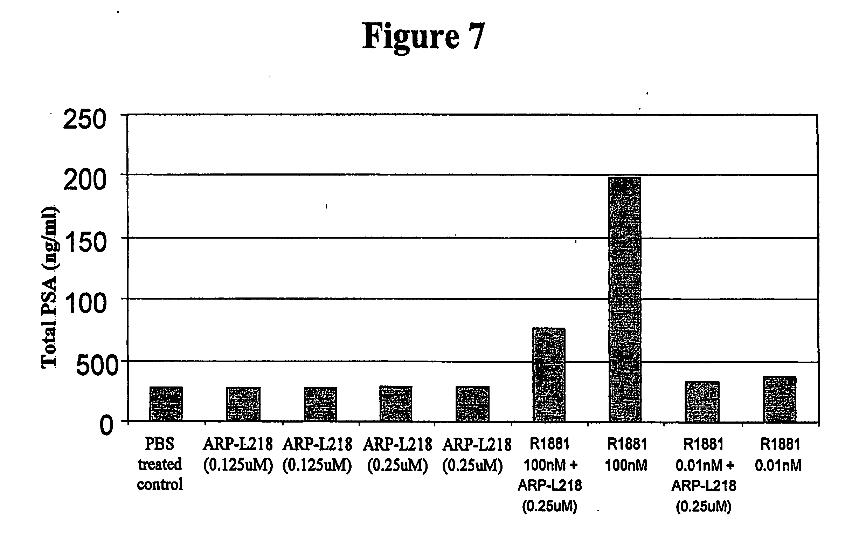

### Figure 7: Down-regulation of PSA protein secretion in LNCap cells by ARP-L218.

Cells were incubated in the presence ofR1881 for 3 days. PSA levels in the supernatants were measure by **ELISA.** Each column represent total prostate specific antigen (PSA).

### Example 1: Construction and use of oligo-regulator peptide fusion molecules.

A series of oligopeptide conjugates useful as gene regulatory molecules has been produced. These consist of at least two specific portions or modules, namely an oligonucleotide capable of forming a DNA triple helix with a selected double-stranded target sequence (Triplex Forming Oligonucleotide, or TFO; Module 1); and a discrete peptide sequence derived from either a gene repressor or activator ( Module 2). The TFO is fused to the repressor or activator peptide.

As an example, the TFO is designed to form a triplex with the Interferon Stimulatable Response Element (ISRE) of the human Interferon Stimulated Gene (ISG) 6-16. (Porter A., et al., EMBO J. 7: 85-92, 1988). The ISRE is very purine rich on one DNA strand and is, therefore, a candidate sequence for forming a DNA triplex by Höogsteen base pairing. The- rules for designing potential TFO are summarised in: Vasquez KM and Wilson JH, Trends Biochem Sci, 1: 4-9, 1998. The sequence 5'-AAAGTAAAAGGGGAGAGAGGG-3' was produced as an oligonucleotide (Module 1) with an activated 5' end for chemical coupling to Module 2 peptides. Module 2 peptides explored in this study include (at least one copy of the minimal transcriptional activator domain of the Herpes Simplex Virus VP16 Transcriptional Activator protein for example the amino acid sequence GGGPADALDDFDLDMLPADALDDFDLDML or GGGPADALDDFDLDMLPADALDDFDLDMLPADALDDFDLDMLPA DALDDFDLDML-CONH₂-(including GGG linker)),and the human MAD1 transcriptional repressor domain (for example amino acids XXXMNIQMLLEAADYLERRERBABHGYASMLP (where XXX is, for example, a AAA or DDD linker)). The latter is a region known to interact with the histone deacetylase complex protein Sin3a. Additionally, we have explored the use of amino acids XXXMAVESRVTQEEIKKEPEKPIDREKTCPLLLRVF (where XXX is, for example, a AAA or DDD linker) of the human Sap 18 protein, also known to associated with Sin3a protein. This region corresponds to a sequence of high evolutionary conservation and overlaps with a region that can mediate gene repression. Module 2 peptides were synthesised in an activated form to enable subsequent coupling to the activated Module 1 oligonucleotide by "native ligation" chemistry (see WO 01/15737 and Stetsenko & Gait (2000) Organic Chem 65(16), 4900-4908), in which an N-terminal thioester-functionalised peptide is coupled to a 5'-cysteinyl oligonucleotide.

Cell lines for transfection work included COS, cells and HT1080 human fibrosarcoma cells. Cells were transiently transfected using a standard liposome based transfection method (or alternatively another delivery method, for example electroporation or microinjection) with an ISRE regulated luciferase reporter gene together with a varying amount of oligopeptide conjugate. This enables a comparison to be made of Interferon dependent luciferase expression with oligopeptide mediated gene regulatory effects. As controls for specificity, cells were also treated with either the oligonucleotide, the peptide or both (ie as separate, unlinked molecules). After suitable times, for example 0.5, 1, 2, 4, 8 and/or 12 hours, the cells were stimulated with IFN and reporter gene activity was measured using a luminometer based assay for luciferase enzyme. Correction for transfection efficiency was determined by the use of a non-interferon dependent GFP control gene.

The ability of the various oligopeptide conjugates to activate or repress transcription of IFN responsive genes was investigated. Delivery of the increasing concentration of fusion molecule oligo-MAD1 represses the reporter gene activity in a concentration dependent manner. Similarly, the delivery of the Sap 18-oligo fusion molecule represses the gene activity. In addition, delivery of oligoVP 16 into the reporter gene-containing cells results in reporter gene activity in the absence of IFN. After the TFO (ie without a peptide domain) was delivered into the cells in addition to an oligo-peptide fusion molecule, the repression of gene activity was less than that seen with the fusion molecule alone, ie repression was equivalent to that seen with a lower concentration of the fusion molecule. This result shows that the molecules with the repressor peptide are more effective regulators of gene activity than the TFO without a peptide domain, and the TFO may compete with the oligo-peptide fusion molecule for binding to the target sequence. The oligo and peptide alone or added together had no repressor effect, demonstrating the specificity of the oligopeptide conjugates in gene regulation.

This example demonstrates the design and construction of fusion molecules consisting of DNA binding oligonucleotides and functional peptides, and their delivery into the cells. The oligopeptide conjugates are able to target specific genes and repressor peptide sequences mediating Gene ICE can repress genes in a specific, targeted manner. Thus, oligopeptide conjugates can be designed to be potent regulators of gene activity.

### Example 2: Repression of chromosomal genes by oligo-regulator fusion molecules

Fusion molecules are able to regulate gene activity, when such genes are integrated into the genome. Fusion molecules containing a DNA binding oligonucleotide (TFO) fused to a MAD1, Sap18 or VP16 peptide were designed and constructed as described in Example 1.

Cells were transfected as described in Example 1 with ISRE-containing reporter genes, and cell lines stably expressing these genes were selected. In these stable cell lines, the gene is integrated in the genome and therefore may function as an endogenous gene.

The fusion molecules were delivered into the cells and experiments were carried out as described in Example 1. Furthermore, the repression was measured at different times in order to establish a time course for repressor effects. The fusion molecules were more effective repressors that TFOs alone. The effect was also specific (for example, the unfused peptide and oligonucleotide did not have the same effect as the oligo-peptide fusion). In addition, the repression by fusion molecules was seen at later time points than any repression seen by the TFO alone, suggesting a more permanent effect. Again, fusion molecule with VP 16 peptide was capable of activating the gene expression.

Thus, the fusion molecules described in example 1 are able to regulate chromosomal gene activity. Fusion molecules with a DNA binding oligonucleotide targeting portion are able to target specific chromosomal genes. A Gene ICE repressor peptide fused to the DNA binding oligonucleotide is able to repress predetermined chromosomal gene activity. Thus, the described fusion molecules are potent regulators of chromosomal gene activity.

Endogenous gene regulation is measured, for example by assessing transcription of the gene (for example using PCR) or by assessing the quantity or activity of the encoded polypeptide. In an example, the oligonucleotide is directed to the Androgen receptor gene regulatory site. In particular, the oligonucleotide has the sequence 5' gggaaaggaaaagaggggaggg 3' or 5' gggaggggaaaggaaaagagg 3'.

In an example, the prostate cancer cell line LnCap is treated with the oligonucleotide-peptide fusion comprising a MAD1 or Sap 18 peptide as described in Example 1. Transfected cells are optionally identified and/or isolated (for example using a GFP marker and FACS techniques) and are assayed for androgen receptor gene expression as well as for the classic prostate cancer marker PSA. For example, GFP-positive, FACS sorted cells were cultured in the presence or absence of the AR agonist R1881. After 72 hours, culture media were collected and the amount of the androgen-regulated protein PSA determined by immunoassay. Addition of R1881 results in an approximately 25-fold increase in levels of secreted PSA in control cells (with no oligonucleotide-peptide fusion, or an irrelevant oligonucleotide-peptide fusion). By contrast PSA levels in test oligonucleotide-peptide transfected cells were raised only about 5-fold, demonstrating an about 5-fold decrease in PSA levels, relative to the control cells. This demonstrates repression of an endogenous gene.

Alternatively, PCR is used to detect and quantify expression and show that the oligonucleotide-peptide fusions repress endogenous androgen receptor gene expression as well as Androgen-regulated gene expression. The Androgen Receptor (AR)-positive human T47D cell line is infected with the test oligonucleotide-peptide fusion (for example labelled with green fluorescent protein (GFP)) or an irrelevant oligaucleotide-peptide fusion (which may also be GFP labelled). Infected cells may be purified by FACS and GFP-positive cells cultured in the presence of the AR ligand R1881 prior to harvesting and preparation of RNA. Expression of the androgen receptor gene as well as androgen-regulated genes PSA and DRG-1 and a non-androgen-regulated gene GAPDH, was determined by PCR. This demonstrates repression of an endogenous gene.

### Example 3: Fusion molecule binding to a target sequence and histone deacetylase complex.

This Example demonstrates that the fusion molecule binds to a specific target sequence as well as to a component of histone deacetylase complex. The fusion molecules containing an oligonucleotide (TFO) and a repressor peptide were produced as described in Example 1.

The different fusion molecules were incubated with labelled oligonucleotide, which was made complementary to the oligo part of a fusion. The same fusion molecules were also incubated with Sin3, which is a component of a histone deacetylation complex. Furthermore, the fusions were also incubated with both the complementary oligonucleotide and Sin3 protein.

The complexes were then analysed by standard band shift analysis methods. The fusion molecules were able to bind to both the labelled complementary oligonucleotide as well as the Sin3 protein, both separately and simultaneously. The unspecific fusions were not able bind the labelled oligo or Sin3, thus demonstrating the specificity of the effect with repressor fusions.

It can be concluded that the repressor fusions can specifically bind their target sequences. The repressor fusions are able to recruit histone deacetylase complexes by binding proteins that are part of this complex, and by binding their target sequences and recruiting the histone deacetylase complexes simultaneously, the described fusion molecules are very potent and specific repressors of gene activity.

### Example 4: Target validation protocol

The available DNA sequence for the gene of interest (including flanking sequence) is analysed in order to select a suitable site for targeting an oligo/peptide to. The oligo/peptide is synthesised and may be tested prior to use in the intended cells or animals or humans, for example using a reporter gene system. The oligo/peptide may be used or tested further in cells *in vitro* or in animals or humans.

Once a gene sequence has been provided, the process will involve:
- The gene of interest (including flanking sequences if necessary) will be scanned for unique sequence elements not found elsewhere in the human genome using bio-informatics data-mining tools (for example the Genetics Computer Group (GCG) program as used in Perkins et al (1998) Biochemistry 37, 11315-11322). A nucleic acid based DNA binding molecule predicted to bind to the identified unique sequence (for example as a TFO) is designed and synthesised.

The DNA binding molecule is likely to be an oligonucleotide, preferably with the following features:
(a) at least 16 nucleotides in length
(b) targeted to a gene promoter or at or near to the transcription initiation site of the gene.

It is preferred that the target site for the binding to a TFO is purine-rich in one strand.

The TFO may be pyrimidine rich (predominantly C or T); purine rich (predominantly G or A) or mixed (predominantly G or T, or G, A or T). CT TFOs are considered to bind in a parallel motif, in which the third strand (TFO) has the same 5' to 3' orientation as the purine strand of the duplex. GA TFOs are considered to bind in an antiparallel motif, in which the TFO is oriented oppositely to the purine strand. Mixed TFOs may bind in a parallel or antiparallel motif, depending on the target sequence. Base pairing arises from formation of Hoogsteen hydrogen bonds in parallel triplexes (T:AT, C⁺:GC and G:GC) and reverse Hoogsteen hydrogen bonds in antiparallel triplexes (G:GC, A:AT and T:AT).

It is intended that the oligonucleotide is a DNA oligonucleotide, possibly with stabilising chemical modifications. Alternative bases, for example N⁶methyl-8-oxo-2-deoxyadenine may be used in place of cytosine, 2-deoxy-6-thioguanine in place of guanine or 7-deaza-2-deoxyxanthine in place of thymine.
- The repressor peptide or peptides may be produced in bulk using a peptide synthesiser and stored frozen until used.
- The repressor peptide-DNA binding molecule construct is prepared and purified. The chemistry used may be that described in WO 01/15737. Kits are available from Link Technologies.
- The construct may be quality controlled by mass spectroscopy and/or by use of labelled complementary oligonucleotide or labelled antibody moieties (using for example fluorescent, chemiluminescent or enzyme labels). Typically in such a method the construct is added to a solid support on which an antibody that binds to the peptide portion of the construct is immobilised and a labelled oligonucleotide that binds to the oligonucleotide portion of the construct is added. In this method detection of the label bound to the solid support demonstrates that the construct is intact. In another typical format the oligonucleotide may be attached to a solid support and the antibody labelled.
- A reporter gene construct may be prepared for the gene of interest (though this is not generally necessary).
- The candidate DNA binding oligonucleotide or oligo/peptide may be tested for the following:
   o Affinity of binding to the target sequence;
   o Specificity of binding by exposure to a whole genome DNA chip.
- The oligo/peptide may be tested for effectiveness using the reporter gene system.

The oligo/peptide may then be used for modulating or suppressing expression of the gene of interest in the cell or animal of interest.

### Example 5: Target validation

The oligo/peptide fusion molecules will be used to validate drug targets. This will involve:
- Carrying out the protocol set out in Example 1.
- Delivery of the construct into cells or tissues. These may be normal or disease tissues, cell lines or primary cells appropriate to the study of the molecule of interest
- Analysis of the phenotype by any expression analysis methods; or any functional analysis such as assessment of cell motility, growth or apoptosis analysis.
- Comparison with any available data for a particular disease and analysis of desired effects such as cell death or motility.

The obtained data will be used to validate the pre-determined drug targets for drug development programmes.

### Example 6: Patient treatment example

A oligo/peptide fusion is produced as described which targets the androgen or estrogen regulated genes. The fusion molecules are prepared in a sterile environment and formulated into liposomes. The fusion-containing liposomes are targeted into the vicinity of breast or prostate. The liposomes are taken up by cancer cells and androgen or estrogen receptor mediated transcription is suppressed selectively in respective cells.

### Example 7: Target identification screen

The oligo/peptide fusion molecules will be used to identify drug targets. This will involve:
- Preparation of a fusion molecule as set out in Example 1
- Delivery of the fusion molecule into the cells or tissues. These may be normal or disease tissues, cell lines or primary cells.
- Analysis of gene expression profile resulting from gene silencing, using DNA arrays. This indicates the effect of the construct on overall gene expression in the model.
- Analysis of the phenotype by any expression analysis methods, or any functional analysis such as cell motility, growth or apoptosis analysis.
The obtained data will be used to find potential drug targets for diseases such as breast or prostate cancer. These targets can be further validated by appropriate methods including any further similar screens, *in vitro* methods and cell and animal models.

### Example 8: Oligo/peptide fusion molecules targeted to the androgen receptor.

As discussed in Example 2, oligo/peptide fusion molecules are able to regulate gene activity when the genes are integrated into the genome. In this example we provide further experimental data supporting this.

We constructed fusions between a DNA binding oligonucleotide (TFO) targeted at the promoter sequence of the androgen receptor gene (ARP), and a peptide containing a 14 or 29 amino acid MAD 1 repressor sequence linked to a 16 amino acid penetratin sequence.

The sequence of the ARP TFO used in this example was:
**ARP TFO**: (5') GFGUGGTGFGGTTGTGTT (3')
U=5-fluro-deoxyuracil
F= 2'-deoxy-6-thioguanine
TFO's are modified at 5' end for conjugation as described by Gait et al (2000) J.Org. Chem, 65, 4900-4908, and at 3' end with standard amino-link to protect from degradation.

The oligonucleotide sequence is designed to form a triplex with the promoter sequence of the androgen receptor.

The TFO was fused to two different peptide fragments. The peptides fragments used in this example were:
**L217:** HHHHHH-Penetratin-DDD-14aaMAD
   (Link)HHHHHHRQIKIWFQNRRMKWKKDDDMNIAMLLEAADYL E(amide)
**L218:** HHHHHH-29aaMAD-DDD-Penetratin
   (Link)HHHHHHMNIAMLLEAADYLERREREAEHGYASMLPDDDR QIKIWFQNRRMKWKK(amide)

The 14 and 29 amino acid peptide sequences are from the transcriptional repressor domain of the MAD1 protein, a region known to interact with the histone deacetylase complex protein Sin3a.
The three Aspartic acid residues (DDD) are a linker sequence, while the Penetratin peptide sequence mediates efficient plasma membrane translocation of the oligo/peptide fusion molecule.

The HIS residues were added in order to provide further purification options.

The experiments were conducted in LNCap (Lymph Node Prostate Carcinoma) human prostate tumour cell line. LNCap cells were obtained from the European Collection of Cell Cultures, accession number B9110211. Cultures were grown and propagated in-house and used as monolayers in disposable tissue culture labware. On the day of testing, cells were observed as having proper cell integrity and therefore, were acceptable for use in this study.

Intra-cellular and intra-nuclear delivery of oligo/peptide fusion molecules to the LnCAP cells was demonstrated using a fluorescent (Cy3) labelled oligo-peptide. The labelled construct (5 pmol in 1ml) and Lipofectamine 2000 (used as per manufacturer's instructions) were added to LnCAP cells and left to incubate for 24 hours. The cells were then examined by fluorescent microscopy. Intra-nuclear localisation of the construct was demonstrated by co-location with a nuclear (DAPI) stain.

The effect of the oligo/peptide fusion molecules ARP-L217 and ARP-L218 on androgen receptor gene expression was measured in the following experiments.

### 1) Treatment of LNCap cells with ARP-L217

To measure the effect of ARP-L217 on the androgen receptor gene expression the following experiments were conducted.

ARP-L217 was prepared in phosphate buffer saline (PBS), 0.2µm syringed filtered and used on day of test set-up.

LNCap cells were transfected with ARP-L217 using lipofectamine2000 (cationic activated dendrimer, InVitrogen life technologies). Various amounts of ARP-L217 were mixed with a fixed amount of lipofectamine2000 (3µl) in a total volume of 100µl serum (10% FBS) supplemented medium without antibiotics. After 20mins incubation at room temperature, the transfection mixture was added to cells at 70% confluency and cells were incubated for 3 days.

RT-PCR conditions were established to give optimal sensitivity within the exponential phase of the amplification process. Total cellular RNA was isolated from the treated LNCap cells using the RNeasy Kit (Qiagen). RNA (1µg of each sample) was reverse transcribed into cDNA using the Omniscript RT kit (Qiagen). The synthesized cDNA (2µl of each sample) was subjected to PCR amplification (Qiagen Taq Kit) with human androgen receptor primers sense 5'-TCCAGAATCTGTTCCAGAGCG-3' and antisense 5'-TTCGGATACTGCTTCCTGC-3' to yield a 281bp product. To verify the quality of RNA/cDNA preparation, PCR amplification was carried out with human β-actin primers (Promega, UK). To verify that PCR product were not amplified from residual DNA left in RNA samples, an RT negative control was subjected to β-actin PCR amplification.

The AR primer sequences used in this study were synthesized by Qiagen-Operon and designed to avoid 5'-3'complementarity and included at least one intron in the product to detect contamination with genomic DNA.

Electrophoresis of PCR products was performed on 2% agarose gels and the gels were pre-casted with ethidium bromide (1:10 dilution). The gels were photographed using the GelDoc system

The comparison and analysis of mRNA expression was done by gel band quantitation using Labworks software system. Sample/β-actin OD ratio was calculated for each sample and then used for comparison. Thus, increased expression of mRNA is presented as a percentage from the control (untreated cells) value.

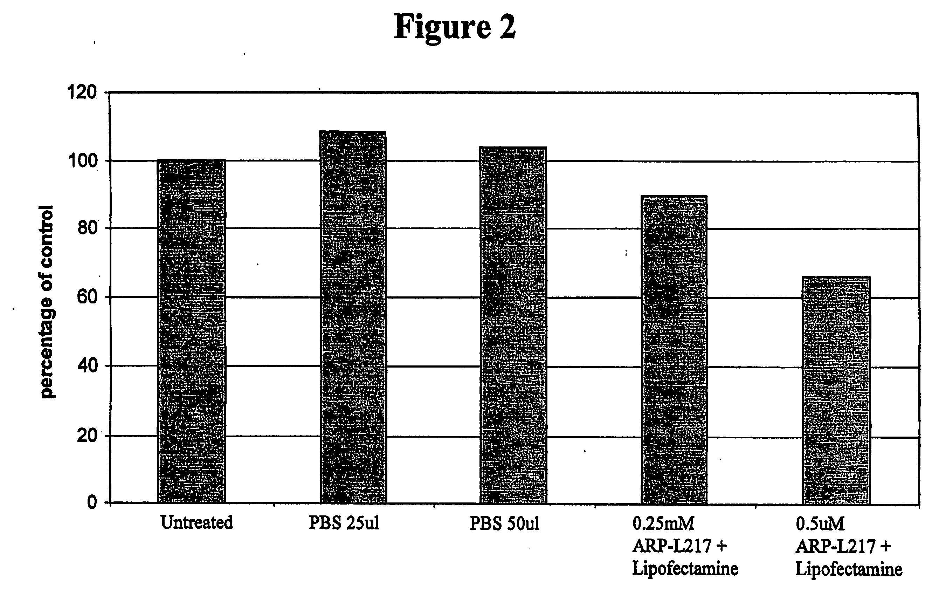

The results from this experiment as shown in Table 1 below and also as a bar graph in Figure 2.

**Table 1a Data illustrating band quantitation after exposure of ARP-L217 to LNCap cells for 3days.**

| | | | | | |
|---|---|---|---|---|---|
| | IODa | IODa | IODb | IODb | Mean |
| **Untreated** | 2280 | 2406 | 2105 | 2162 | 2238.25 |
| **PBS 25ul** | 2283 | 1908 | 2106 | 2544 | 2210.25 |
| **PBS 50ul** | 1799 | 1799 | 2677 | 2268 | 2135.75 |
| O25µM ARP-L217+Lipofectamine | 1428 | 1749 | 1419 | 1325 | 1480.25 |
| 0.5µMARP-L217-Lipofectamine | 783 | 971 | 1037 | 1191 | 995.5 |

| | | β-actin PCR Band Qua | | | ntitation (Day 3) |
|---|---|---|---|---|---|
| | IODa | IODa | IODb | IODb | Mean |
| Untreated | 8957 | 9589 | 8996 | 9663 | 9301.25 |
| **PBS 25ul** | 8228 | 7980 | 9098 | 8544 | 8462.5 |
| PBS 50ul | 7413 | 8762 | 8920 | 9005 | 8525 |
| 0.25µM ARP-L217+LIpofectamine | 6632 | 6322 | 6605 | 7924 | 6870.75 |
| 0.5uM ARP-L217+Lipofectamine | 6323 | 6180 | 5834 | 6695 | 6258 |

**Table 1b Determination of ratio per beta-actin and percentage inhibition from control.**

| | **Ratio/ß-actin** | **Percentage from untreated control** |
|---|---|---|
| Untreated | 0.2406 | 100 |
| PBS 25ul | 0.2611 | 108.5364073 |
| PBS 50ul | 0.2505 | 104.1091143 |
| 0.25µM ARP-L217+Lipofectamine | 0.2154 | 89.52898226 |
| 0.5uM ARP-L217+Lipofectamine | 0.1590 | 66.10562717 |

From this data it can be seen that the cell samples which were incubated with ARP-L217 have greatly reduced androgen receptor gene expression levels compared with those treated with control PBS solutions.

### 2) Treatment of LNCap cells with ARP-L218 and R1881

The effect of ARP-L218 on androgen receptor gene expression with and without a synthetic androgen (R1881) was measured using the protocol outlined in the above section.

ARP-L218 was prepared in phosphate buffer saline (PBS), 0.2µm syringed filtered and used on day of test set-up. R1881 (synthetic androgen) was purchased from Perkin Elmer, Catalogue number NLP005005MG.

LNCap cells were transfected with ARP-L218 using lipofectamine2000 (cationic activated dendrimer, InVitrogen life technologies) with/without agonist. Various amounts of ARP-L218 were mixed with a fixed amount of lipofectamine2000 (3µl) in a total volume of 100µl serum free medium without antibiotics (OptiMEM). After 20mins incubation at room temperature, the transfection mixture was added to cells at 70% confluency and cells were incubated for 3 days.

Intra-cellular and intra-nuclear delivery of oligo/peptide fusion molecules to the LnCAP cells was demonstrated using a fluorescent (Cy3) labelled oligo-peptide. The labelled construct (5 pmol in 1ml) and Lipofectamine 2000 (used as per manufacturer's instructions) were added to LnCAP cells and left to incubate for 24 hours. The cells were then examined by fluorescent microscopy. Intra-nuclear localisation of the construct was demonstrated by co-location with a nuclear (DAPI) stain.

RNA was extracted and RT-PCR analysis of AR mRNA levels conducted as described above.

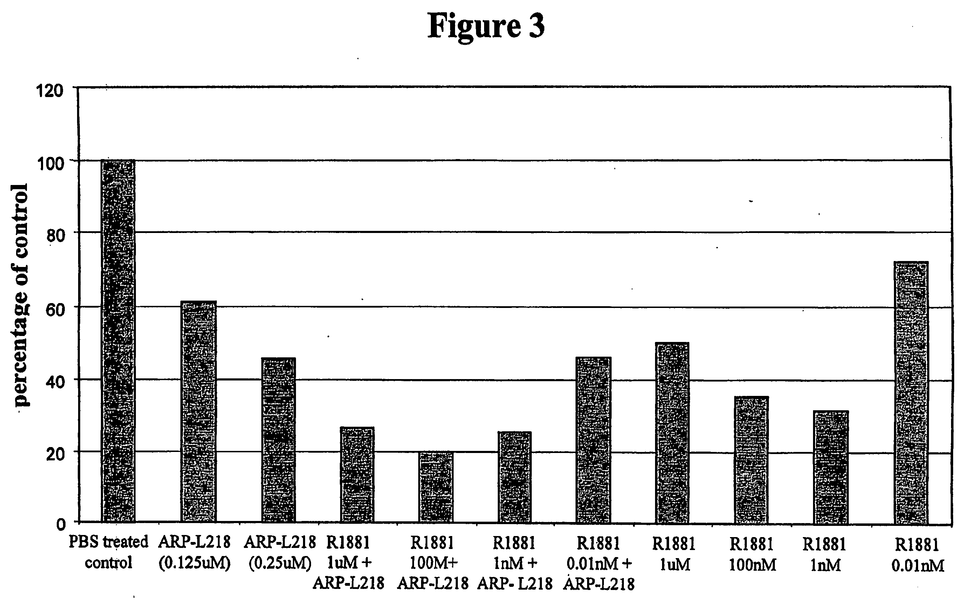

The results from this experiment as shown in Table 2 below and also as a bar graph in Figure 3

**Table 2: Data illustrating band quantitation after exposure of ARP-L218 in presence/absence of synthetic androgen (R1881) to LNCap cells for 3days. Determination of ratio per beta-actin and percentage inhibition from control.**

| Sample ID | AR | AR | Average | beta-actin | beta-actin | Average | Ratio/beta-actin | Percentage of control |
|---|---|---|---|---|---|---|---|---|
| PBS treated control | 3142 | - | 3142 | 9521 | - | 9521 | 0.330 | 100 |
| ARP-L218 (0.125uM) | 2327 | 2647 | 2487 | 10592 | 14178 | 12385 | 0.201 | 60.84 |
| ARP-L218 (0.25uM) | 2728 | 2212 | 2470 | 17103 | 15691 | 16397 | 0.151 | 45.64 |
| R1881 1uM + ARP-L218 | 1291 | - | 1291 | 14949 | - | 14949 | 0.086 | 26.16 |
| R1881 100nM + ARP-L218 | 1068 | - | 1068 | 16258 | - | 16258 | 0.066 | 19.90 |
| R1881 1nM + ARP-L218 | 1393 | - | 1393 | 16950 | - | 16950 | 0.082 | 24.90 |
| R1881 0.01nM + ARP-L218 | 2532 | - | 2532 | 16607 | - | 16607 | 0.152 | 46.20 |
| R1881 1uM | 1388 | - | 1388 | 8424 | - | 8424 | 0.165 | 49.92 |
| R1881100nM | 1455 | - | 1455 | 12536 | - | 12536 | 0.116 | 35.17 |
| R1881 1nM | 1471 | - | 1471 | 14242 | - | 14242 | 0.103 | 31.29 |
| R1881 0.01 nM | 2083 | - | 2083 | . 8799 | - | 8799 | 0.237 | 71.73 |

From this data it can be seen that the cell samples which were incubated with ARP-L218 and R1881 have greatly reduced androgen receptor gene expression levels compared with those treated with R1881-containing solutions. Both types of samples had reduced AR mRNA levels than the control cell sample treated with PBS.

Therefore while R1881 can act to reduce AR gene expression this reduction can be enhanced by coincubating the cells with the ARP-L218 oligo/peptide fusion molecule.

### 3) Treatment of LNCap cells with ARP-L218 and cyproterone acetate

The effect of ARP-L218 with and without cyproterone acetate, a receptor bound androgen receptor antagonist, on androgen receptor gene expression was measured using the protocol outlined in the above section.

ARP-L218 was prepared in phosphate buffer saline (PBS), 0.2µm syringed filtered and used on day of test set-up. Cyproterone acetate was purchased from Sigma, lot number 41K1195.

LNCap cells were transfected with ARP-L218 using lipofectamine2000 (cationic activated dendrimer, InVitrogen life technologies) with/without antagonist. Various amounts of ARP-L218 were mixed with a fixed amount of lipofectamine2000 (3µl) in a total volume of 100µl serum free medium without antibiotics (OptiMEM). After 20mins incubation at room temperature, the transfection mixture was added to cells at 70% confluency and cells were incubated for 3 days.

Intra-cellular and intra-nuclear delivery of oligo/peptide fusion molecules to the LnCAP cells was demonstrated using a fluorescent (Cy3) labelled oligo-peptide. The labelled construct (5 pmol in 1ml) and Lipofectamine 2000 (used as per manufacturer's instructions) were added to LnCAP cells and left to incubate for 24 hours. The cells were then examined by fluorescent microscopy. Intra-nuclear localisation of the construct was demonstrated by co-location with a nuclear (DAPI) stain.

RNA. was extracted and RT-PCR analysis of AR mRNA levels conducted as described above.

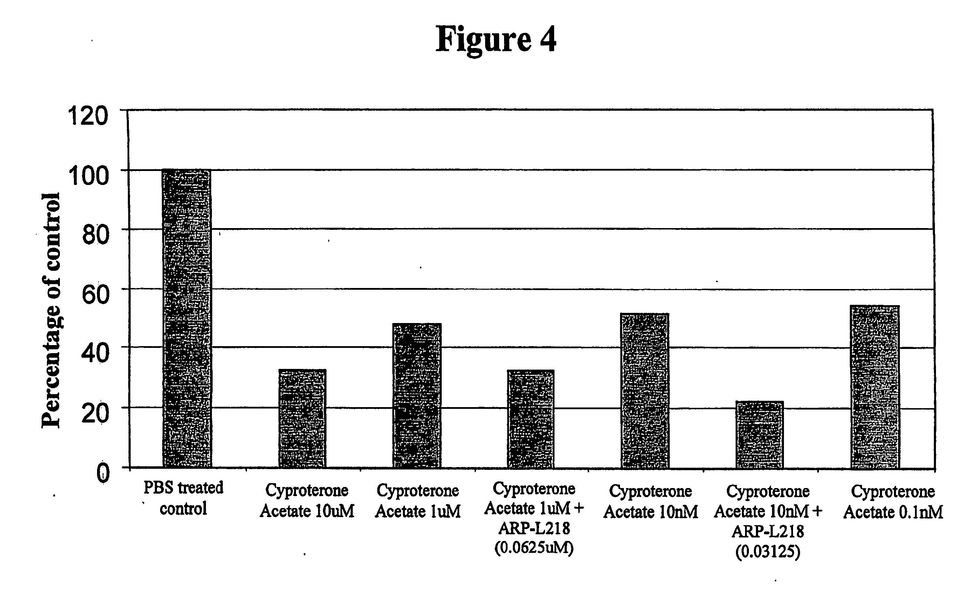

The results from this experiment as shown in Table 3 below and also as a bar graph in Figure 4

**Table 3: Data illustrating band quantitation after exposure of ARP-L218 in presence/absence of androgen receptor antagonist (cyproterone acetate) to LNCap cells for 3 days. Determination of ratio per beta-actin and percentage inhibition from control.**

| | | | | |
|---|---|---|---|---|
| **sample ID** | **AR** | **beta-actln** | **Ratio** | **% of control** |
| PBS treated control | 3142 | 9521 | 0.330 | 100 |
| Cyproterone Acetate 10uM | 1568 | 14691 | 0.107 | 32.4 |
| Cyproterone Acetate 1uM | 1868 | 11856 | 0.158 | 47.8 |
| Cyproterone Acetate 1uM + ARP-L218(0.0625uM) | 1583 | 14861 | 0.107 | 32.4 |
| Cyproterone Acetate 10nM | 2401 | 14112 | 0.170 | 51.5 |
| Cyproterone Acetate 10nM + ARP-L218 (0.03125) | 1241 | 16885 | 0.074 | 22.4 |
| Cyproterone Acetate O.1nM | 2357 | 13278 | 0.178 | 54.0 |

From this data it can be seen that the cell samples which were incubated with ARP-L218 and cyproterone acetate have reduced androgen receptor gene expression levels compared with those treated with a cyproterone acetate only. Both types of samples had reduced AR mRNA levels compared with the control cell sample treated with PBS.

Therefore while cyproterone acetate can act to reduce AR gene expression this reduction can be enhanced by coincubating the cells with the ARP-L218 oligo/peptide fusion molecule.

### Example 9: Down regulation of an interferon stimulated, genome incorporated gene.

As discussed in Example 2, oligo/peptide fusion molecules are able to regulate gene activity when the genes are integrated into the genome. In this example we provide further experimental data supporting this.

We constructed fusions between a DNA binding oligonucleotide (TFO) targeted to an interferon stimulated response element (IRE), and a peptide containing a 29 amino acid MAD 1 repressor sequence linked to a 16 amino acid penetratin sequence.

The sequence of the IRE TFO used in this example was:
**IRE TFO:** (5') GGGUGGTGGGGTTGTGTT (3')
U=5-fluro-deoxyuracil
TFO's are modified at 5' end for conjugation as described by Gait et al (2000) J.Org. Chem, 65, 4900-4908, and at 3' end with standard amino-link to protect from degradation.

The oligonucleotide sequence is designed to form a triplex with the Interferon Stimulatable Response Element (ISRE) of the human Interferon Stimulated Gene (ISG) 6-16 (Porter et al., (1988) EMBO J, 7, 85-92).

The TFO was fused to two different peptide fragments. The peptides fragments used in this example were:
**L218:** HHHHHH-29aaMAD-DDD-Penetratin
   (Link)HHHHHHMNIAMLLEAADYLERREREAEHGYASMLPDDDR QIKIWFQNRRMKWKK(carboxamide)
**L219:** DDD-29aaMAD-HHHHHH-Penetratin
   (Link)DDDMNIAMLLEAADYLERREREAEHGYASMLPHHHHHHR QIKIWFQNRRMKWKK (carboxamide)

The 29 amino acid peptide sequence is from the transcriptional repressor domain of the MAD1 protein, a region known to interact with the histone deacetylase complex protein Sin3a.

The three Aspartic acid residues (DDD) are a linker sequence, while the Penetratin peptide sequence mediates efficient plasma membrane translocation of the oligo/peptide fusion molecule.

The HIS residues were added in order to provide further purification options.

Modified HT1080 human fibrosarcoma cells were used to demonstrate the oligo/peptide fusion molecules capacity to downregulate an interferon stimulated reporter gene expression. These cells have a stably incorporated gene expressing EGFP under the control of the interferon stimulated response element (IRE) of the human 6-16 gene.

For the following data, cells were transiently transfected with different compounds using standard lipofectamine protocols. Cells were then treated with 300 units/ml of interferon (INF) for one day and analysed by FACS for the GFP expression.

Figure 5 shows the FACS analysis of a number of different cell populations, in which the following experimental conditions were applied:
a) Parental cell line, no treatment
b) Cells expressing the stably transfected construct (C8), no treatment
c) C8 treated with interferon (+INF)
d) C8 transfected with 500 pm TFO, +INF
e) C8 specific TFO 1000pM, +INF
f) C8, IRE-L218 500pM, +INF
g) C8, IRE-L219 500pM, +INF
h) C8, unspecific control TFO 500pM, +INF
i) C8, unspecific control TFO 1000pM, +INF

Table 4 shows the interferon-induced GFP expression of Figure 5 presented as percentage.

**Table 4: Interferon-induced GFP expression.**

| Condition applied | GFP expression |
|---|---|
| (a) | 0% |
| (b) | <1% |
| (c) | 90% |
| (d) | 53% |
| (e) | 63% |
| (f) | 40% |
| (g) | 42% |
| (h) | 87% |
| (i) | 85% |

The IRE - TFO system, which was used as a model system, has been previously described (Roy, 1994, Eur. J. Biochem. 220, 493-503). The results from FACS analysis show that IRE-L218 and IRE-L219 were able to repress the expression by an average of 59%, whereas the specific TFO of the same sequence performed an average 42% repression. The control TFOs had only marginal effect over the positive control.

These results clearly illustrate that the oligo/peptide fusion molecules are most effective in repressing the INF induced GFP gene expression.

### Example 10: Down-regulation of gene expression by oligo/peptide fusion molecules is associated with chromatin histone deacetylation

As seen in Examples 8 and 9 the oligo/peptide fusion molecules are effective in repressing gene expression. We propose that this is due to a MAD1-mediated change in the histone acetylation state of DNA at or close to where the ARP or IRE oligo binds.

To show that reduced gene expression is associated with chromatin histone deacetylation, the Chromatin immunoprecipitation (ChIP) method may be used. Chromatin immunoprecipitation is carried out using a ChIP Assay kit according to the manufacturer's instructions (Upstate Biotechnology, Bucks, UK).

LNCap human prostate tumour cells are grown and propagated and incubated with oligo/peptide fusion molecules which are effective in repressing androgen receptor gene expression. Untreated LNCap cells are used as a control.

Cells are grown to 95% confluence on 35cm tissue culture plates in DMEM, lacking phenol red, supplemented with 5% DSS, P/S/G and G418 (100µg/ml). Hygromycin B (80µg/ml) and doxycycline (1µg/ml) are added as appropriate. 30 minutes prior to fixation, E2 (10-8M) or ethanol (as a control) is added to the cells. 37% formaldehyde is added dropwise directly to the medium to a final concentration of 1%. Cells are incubated for 10 minutes at 37°C.

On ice, the medium is aspirated from the plates, cells are washed twice with ice cold PBS containing 1x protease inhibitors (PI) (Sigma, Dorset, UK). For harvesting, 1ml of ice cold PBS containing 1x PI is added to the plate and the cells scraped into pre-cooled microfuge tubes, using a rubber policeman. Cells are pelleted by centrifugation at 2000rpm for 4 minutes, at 4°C. The pellets are resuspended in 400µl of warmed ChIP SDS-lysis buffer (1% SDS; 10mM Na EDTA pH 8.0; 50MM Tris-HCl pH 8.1) containing PI, and incubated on ice for 10 minutes.

The lysates are sonicated to shear the DNA into 200 - 1000bp lengths. During sonication, the samples are placed in an ice-water beaker, to keep them cold in order to prevent sample degradation. Sonication is carried out using a Soniprep 150 sonicator with attached Soniprep 150 exponential titanium probe (Sanyo-Gallenkamp, Leics, UK) with four 10 second bursts, separated by 30 second intervals.

Samples are centrifuged at 13,000 rpm for 10 minutes at 4°C. The supernatant is collected into 15ml sterile falcon tubes and diluted 10 fold with 3600µl ChIP dilution buffer (0.01% SDS; 1.1% Triton-X-100; 1.2mM Na EDTA pH 8.0; 16.7mM Tris-HCl, pH 8.1; 167mM NaCl). The samples are then divided into two 2ml aliquots in 2.5ml tubes, one for incubation with an anti-acetylated histone H4 antibody, ChIPs grade (Upstate Biotechnology, Bucks, UK), and the other for use as a no antibody control.

To reduce non-specific background, each 2ml aliquot is pre-cleared by adding 80µl of salmon sperm DNA/protein A agarose-50% slurry (suspended in 10mM Tris-HCl pH 8.0; 1mM Na EDTA pH 8.0) for 30 minutes at 4°C on a vertical rotating platform (Stuart Scientific, Staffs, UK). The agarose beads are then pelleted by a 30-second centrifugation at 1000 rpm and the supernatant fractions collected into fresh 2.5ml tubes. The immunoprecipitating antibody is added at a dilution of 1:500 to the first sample but not to the no antibody control sample. Both tubes are incubated overnight at 4°C on a vertical rotating platform (Stuart Scientific, Staffs, UK).

60µl of salmon sperm DNA/protein A Agarose-50% slurry are incubated with each tube for 1 hour at 4°C, with rotation, to collect the antibody/histone complexes or non-specifically bound proteins in the case of the no antibody control. The agarose beads are pelleted by brief centrifugation at 800 rpm for 1 minute. The supernatants are carefully transferred into fresh 2.5ml tubes and stored at -20°C.

The protein A Agarose beads/antibody/histone complex is washed for 5 minutes on a vertical rotating platform at 4°C with 1ml of each of the following buffers in the order listed below:
(a) Low Salt Immune Complex Wash Buffer-one wash (0.1% SDS; 1% Triton-X-100; 2mM Na EDTA pH 8.0; 20mM Tris-HCl pH 8.1; 150mM NaCl)
(b) High Salt Immune Complex Wash Buffer - **one wash** (0.1 % SDS; 1% Triton-X 100; 2mM Na EDTA pH 8.0; 20mM Tris-HCl pH 8.1; 500mM NaCl)
(c) LiCl Immune Complex Wash Buffer - **one wash** (0.25M LiCl; 1% NP40 (nonidet); 1% deoxycholate; 1mM Na EDTA pH 8.0; 10mM Tris-HCl pH8.1)
(d) 1x TE **- two washes** (10mM Tris-HCl, 1mM Na EDTA pH 8.0)

The histone/immune complex is eluted from the agarose beads by addition of 250µl freshly prepared Elution buffer (1% SDS; 0.1M NaHCO₃). The samples are vortexed briefly to mix and incubated at room temperature for 15 minutes on a vertical rotating platform. The beads are centrifuged at 1000 rpm for 2 minutes at room temperature and the eluate transferred to fresh microfuge tubes. The elution step is repeated with a further 250µl of Elution buffer and the eluates combined.

20µl of 5M NaCl are added to the eluates and histone-DNA crosslinks reversed by heating to 65°C for at least 4 hours. 10µl of 0.5M Na EDTA pH 8.0,20µl of 1M Tris-HCl, pH 6.5 and 2µl of 10mg/ml Proteinase K are added to the eluted samples. The crosslinks are also reversed on the supernatant fraction from the IP. 40µl of 0.5M Na EDTA pH 8.0, 80µl of 1M Tris-HCl, pH 6.5 and 8µl of 10mg/ml Proteinase K are added to supernatant samples. Samples are incubated for 1 hour at 45°C to degrade protein in the samples.

20µg of glycogen are added to the samples as an inert carrier and then the sample DNAs are recovered by phenol/chloroform/isoamyl alcohol extraction and ethanol precipitation. DNA pellets are resuspended in 50µl sterile water for PCR reactions. 1µl of sample and 35-40 cycles are used for each PCR amplification. Computer-based image analysis (NIH image analysis program) is employed to evaluate the relative levels of the estrogen-regulated androgen receptor gene PCR product, compared with the ß-actin and no antibody controls. This allowed a calculation of the relative amount of the gene transcript contained within a sample to be compared with that in other samples.
Oligonucleotides used for ChIP analysis:
Progesterone Receptor Forward primer:
5'- TCCAGAATCTGTTCCAGAGCG -3'
Progesterone Receptor Reverse primer.
5'- TTCGGATACTGCTTCCTGC -3'
β -actin Forward primer. 5'-TTTTCGCAAAAGGAGGGGAG-3'
β-actin Reverse primer. 5'-AAAGGCAACTTTCGGAACGG-3'

An example of the type of result that may be achieved is shown in Figure 6.

The amount of precipitated androgen receptor DNA, and hence the degree of acetylation of the chromatin histone proteins associated with the androgen receptor, may be decreased by approximately 75% in the cells in which androgen receptor expression is inhibited by the described method compared to untreated cells.

### Example 11: Gel shift assay

The previous examples have demonstrated that the ARP-L217 and ARP-L218 oligo/peptide fusion molecules are able to regulate targeted gene activity. This is considered to be due to a MAD1-mediated change in the histone acetylation state of DNA at or close to where the ARP or IRE oligo binds.

A gel shift assay is used to demonstrate that the oligo/peptide fusion molecules can bind to DNA fragment containing the target promoter.

A 281bp androgen receptor DNA fragment containing the target promoter sequence is incubated with ARP-L217. Samples are subjected to non-denaturing gel electrophoresis in order to characterize triplex-mediated photoadduct. Adducts are detected in samples containing the ARP-L217 product which shifted with increasing doses.

In this way it is possible to show that oligo/peptide fusion molecules can bind to DNA fragment containing the target promoter.

### Example 12: Oligo/peptide fusion molecules with a nuclear localisation signal.

The peptide portion of the molecule may have a nuclear localisation signal (NLS) to target the molecule to the nucleus. The peptides used in this example are:
a) DDD-MAD1-DDD-NLS,
   which has the amino acid sequence:
   (Link)DDDMNIQMLLEAADYLERREREAEHGYASMLPDDDPKKKRK V (carboxamide)
   and,
b) DDD-NLS-DDD-MAD1,
   which has the amino acid sequence:
   (Link)DDDPKKKRKVDDDMNIQMLLEAADYLERREREAEHGYASML P (carboxamide)

The NLS is a 7 amino acid (sequence PKKKRKV) functional nuclear localisation signal derived from the SV40 T-antigen.

The DDD linker sequence and 29 amino acid MAD1 amino acid sequence are the same as those discussed in the earlier examples.

To demonstrate that the DDD-MAD1-DDD-NLS, and DDD-NLS-DDD-MAD1 peptide sequences are targeted to the nucleus, A459 human lung carcinoma cells were transfected using with GeneICE oligopeptides consisting of both the DDD-MAD1-DDD-NLS, and DDD-NLS-DDD-MAD1 peptide sequences linked to a Cy3 labelled oligonucleotide by standard Lipofectamine 2000 protocols. The cells were then fixed in formaldehyde and stained with DAPI nuclear stain using the manufacturer's recommended procedure. Examination of the cells by fluorescence microscopy showed that the Cy3 labelled GeneICE oligopeptide molecule was co-localised with the nuclear DAPI stain. From the resulting experimental data it was concluded that the NLS is very effective at targeting the peptide to the nucleus.

The DDD-MAD1-DDD-NLS, and DDD-NLS-DDD-MAD1 peptide sequences may mediate target gene expression when incorporated into an oligo/peptide molecules of the invention. This can be demonstrated using the experimental approach outlined in Examples 8 and 9.

For example, DDD-MAD1-DDD-NLS, and DDD-NLS-DDD-MAD1 peptide sequences can be incorporated into the oligo/peptide molecules:
ARP:-DDD-MAD1-DDD-NLS, and
ARP:-DDD-NLS-DDD-MAD1.

ARP is the TFO oligo sequence shown in Example 8.

The ARP:-DDD-MAD1-DDD-NLS and ARP:-DDD-NLS-DDD-MAD1 molecules are then transfected into LNCap cells, as in Example 8, or modified HT1080 cells, as in Example 9. Using the experimental procedures outlined in those sections it is possible to show the effect of the ARP:-DDD-MAD1-DDD-NLS and ARP:-DDD-NLS-DDD-MAD1 molecules on target gene expression.

### Example 13: Regulation of prostate specific antigens levels using oligo/peptide fusion molecules.

As discussed in Examples 2,8 and 9 oligo/peptide fusion molecules are able to regulate gene activity when the genes are integrated into the genome. In this example we provide further experimental data supporting this.

Prostate specific antigens (PSA) protein levels are regulated by the androgen receptor protein (AR). Since we have shown in Example 8 that AR mRNA levels are reduced in cells transfected with the ARP-L218 oligo/peptide fusion molecules, we reasoned that there should be a decrease in PSA protein levels in cells transfected with ARP-L218.

Therefore, using the protocols described in Example 8 ARP-L218 was transfected into LNCap cells (GET/LNC/W39/P6) with Lipofectamine 2000 (InVitrogen) with or without R1881 (Perkin Elmer), a synthetic androgen, for 3 days, after which the supernatant were harvested for quantitation of total PSA protein at the Pathology Centre, Hammersmith Hospital, London, U.K.

PSA measurements were carried out by a chemiluminescent labelled immunometric assay using an automated immunoassay analyser (Abbott Architect Immunoassay Analyser).

The results from this experiment are shown in Table 5 below and also as a bar graph in Figure 7.

**Table 5: Total PSA quantified from cell supernatants**

| **Sample Identification** | **Total PSA(ng/ml)** |
|---|---|
| PBS treated control | 279 |
| ARP-L218 (0.125uM) | 279 |
| ARP-L218 (0.125uM) | 270 |
| ARP-L218 (0.25uM) | 287 |
| ARP-L218 (0.25uM) | 282 |
| R1881 100nM + ARP-L218 (0.25uM) | 757 |
| R1881 100nM | 1980 |
| R1881 0.01 nM + ARP-L218 (0.25uM) | 330 |
| R1881 0.01 nM | 372 |

From this data it can be seen that cells which were incubated with the synthetic androgen R1881 show an increase in PSA levels, as would be expected. However, when the cells are transfected with R1881 and ARP-L218, the increase in PSA levels is greatly reduced. As can be seen from the samples transfected with ARP-L218 but not R1881, ARP-L218 has no direct effect on PSA levels.

Therefore, we propose that R1881 acts to increase AR activity which, in turn, leads to an increase in PSA levels. However, ARP-L218 acts to block AR gene expression (as is shown in Example 8), and so PSA levels are subsequently reduced. The data suggests that the oligo/peptide fusion molecules of the invention can be used to directly or indirectly regulate a target gene.

## Claims

1. A molecule for suppressing the expression of a selected gene in a cell comprising (1) a nucleic acid binding portion which binds to a site at or associated with a selected gene which site is present in a genome and (2) an expression repressor portion, wherein the nucleic acid binding portion comprises an oligonucleotide or oligonucleotide mimic or analogue, and wherein the repressor portion comprises a polypeptide or peptidomimetic.

2. A molecule for modulating the expression of a selected gene in a cell comprising (1) a nucleic acid binding portion which binds to a site at or associated with a selected gene which site is present is a genome and (2) a modifying portion, wherein the nucleic acid binding portion comprises an oligonucleotide or oligonucleotide mimic or analogue, and wherein the modifying portion comprises a polypeptide or peptidomimetic which is capable of modulating covalent modification of nucleic acid or chromatin and is not an endonuclease or KRAB.

3. The molecule of Claim 1 or 2 wherein the repressor or modifying portion is a chromatin inactivation portion and is not KRAB.

4. The molecule of Claim 1 or 2 wherein the repressor or modifying portion is all or a portion of a component of a DNA methylase complex or all or a portion of a polypeptide which binds to or facilitates the recruitment of a DNA methylase complex and is not KRAB.

5. The molecule of Claim 1 or 2 wherein the repressor or modifying portion is all or a portion of a component of a histone acetyltransferase or all or a portion of a polypeptide which binds to or facilitates the recruitment of a histone acetyltransferase complex and is not KRAB.

6. The molecule according to any one of the preceding claims wherein the polypeptide or peptidomimetic part of the molecule has a molecular mass of less than 11 kDa.

7. A molecule according to any one of the preceding claims wherein the nucleic acid binding portion is a DNA binding portion.

8. A molecule according to any one of Claims 1 to 6 wherein the nucleic acid binding portion is an RNA binding portion and the site present in a genome is a nascent RNA being transcribed from DNA.

9. The molecule of any of the preceding claims wherein the oligonucleotide or olignucleotide analog or mimetic is a triple forming oligonucleotide (TFO).

10. The molecule of any of the preceding claims wherein the oligonucleotide analog or mimetic is a peptide nucleic acid (PNA).

11. A molecule according to Claim 3 or claims dependent thereon wherein the chromatin inactivation portion facilitates histone deacetylation.

12. A molecule according to Claim 3 or claims dependent thereon or 11 wherein the chromatin inactivation portion is all or a portion of a component of a histone deacetylation (HDAC) complex or all or a portion of a polypeptide which binds to or facilitates the recruitment of a HDAC complex.

13. A molecule according to Claim 12 wherein the component of the HDAC complex or the polypeptide which binds to or facilitates the recruitment of a HDAC complex is any one of PLZF, N-CoR, SMRT, Sin3, SAP 18, SAP30, HDAC, NuRD, MAD1, MAD2, MAD3, MAD4, Rb or E7.

14. A molecule according to Claim 13 wherein the chromatin inactivation portion is all or a N-CoR- or SMRT-binding part of PLZF.

15. A molecule according to Claim 13 wherein the chromatin inactivation portion is all or an enzymatically active part of a HDAC.

16. A molecule according to Claim 13 wherein the chromatin inactivation portion is all or a histone deacetylase complex-binding part of E7.

17. A molecule according to any of the preceding claims wherein the molecule further comprises a portion which facilitates cellular entry and/or nuclear localisation.

18. A molecule according to Claim 17 wherein the portion which facilitates cellular entry and/or nuclear localisation is a small peptide of 7-16 amino acids, for example Modified Antennapedia homeodomain according to the sequence RQIKIWFQNRRMKWKK or basic HIV TAT internalisation peptide according to the sequence (Acm) GRKKRRQRRRPQC, where C(Acm) is a Cys-acetamidomethyl.

19. A molecule according to any one of Claims 1 to 18 wherein the nucleic acid binding portion and the repressor or modifying portion are fused.

20. Use of a molecule according to any of claims 1 to 19 in the manufacture of an agent for modulating the expression of the selected gene in a cell.

21. The use of Claim 20 wherein the agent is for suppressing the expression of the selected gene.

22. The use according to Claim 20 or 21 wherein the agent is a medicament for modulating or suppressing the expression of a selected gene in an animal, wherein the animal has cancer.

23. Use of a molecule according to any of claims 1 to 19 in the manufacture of a medicament for suppressing the expression of a selected gene in a patient who has cancer.

24. A molecule according to any of claims 1 to 19 for the use in medicine.

25. A pharmaceutical composition comprising a molecule according to any of claims 1 to 19 and a pharmaceutically acceptable carrier.

26. The composition of Claim 25 comprising means for promoting cellular uptake of the molecule.

27. The composition of Claim 26 wherein the means for promoting cellular uptake of the molecule is lipsomes or a viral carrier.

28. A host cell comprising a molecule according to any of claims 1 to 19.

29. A host cell according to Claim 28 which is a bacterial cell.

30. A host cell according to Claim 28 which is a animal cell.

31. A host cell according to Claim 28 which is a plant cell.

32. A non-human animal comprising a host cell according to Claim 30.

33. A plant comprising a host cell according to Claim 31.

34. A method for designing a molecule for suppressing expression of a selected gene in a cell, the method comprising
(1) identifying a site at or associated with the selected gene.
(2) identifying or designing a nucleic acid binding portion which binds to, or is predicted to bind to, the site
(3) preparing a molecule comprising the nucleic acid binding portion and an expression repressor portion,
wherein the nucleic acid binding portion comprises an oligonucleotide or oligonucleotide mimic or analogue, and wherein the repressor portion comprises a polypeptide or peptidomimetic.

35. A method for designing a molecule for modulating expression of a selected gene in a cell, the method comprising
(1) identifying a site at or associated with the selected gene
(2) identifying or designing a nucleic acid binding portion which binds to, or is predicated to bind to, the site
(3) preparing a molecule comprising the nucleic acid binding portion and a modifying portion,
wherein the nucleic acid binding portion comprises an oligonucleotide or oligonuleotide mimic or analogue, and wherein the modifying portion comprises a polypeptide or peptidomimetic which is capable of modulating covalent modification of nucleic acid or chromatin.

36. A method for designing a molecule for suppressing expression of a selected gene in a cell, the method comprising
(1) identifying a site at or associated with the selected gene
(2) identifying or designing a nucleic acid binding portion which binds to, or is predicted to bind to, a polynucleotide having or comprising the nucleotide sequence of the site
(3) preparing a molecule comprising the nucleic acid binding portion and an expression repressor portion.
wherein the nucleic acid binding portion comprises an oligonucleotide or oligonucleotide mimic or analogue, and wherein the repressor portion comprises a polypeptide or peptidomimetic

37. A method for designing a molecule for modulating expression of a selected gene in a cell, the method comprising
(1) identifying a site at or associated with the selected gene
(2) identifying or designing a nucleic acid binding portion which binds to, or is predicted to bind to, a polynucleotide having or comprising the nucleotide sequence of the site
(3) preparing a molecule comprising the nucleic acid binding portion and a modifying portion.
wherein the nucleic acid binding portion comprises an oligonucleotide or oligonucleotide mimic or analogue, and wherein the modifying portion comprises a polypeptide or peptidomimetic which is capable of modulating covalent modification of nucleic acid or chromatin.

38. The method of Claim 34 or 35 further comprising of steps of
(4) performing a quality control assessment on the molecule preparation in order to determine that the nucleic acid binding portion and repressor or modifying portion are attached to each other; and/or
(5) testing the affinity and/or specificity of binding of the nucleic acid binding portion to the site; and/or
(6) testing the affinity and/or specificity of binding of the molecule to the site; and/or
(7) testing the efficacy of the molecule or polynucleotide in modulating or suppressing the expression of the gene and/or of a reporter gene comprising the nucleotide sequence of the site.

39. The method of Claim 36 or 37 further comprises of steps of
(4) performing a quality control assessment on the molecule preparation in order to determine that the nucleic acid binding portion and repressor or modifying portion are attached to each other; and/or
(5) testing the affinity and/or specificity of the binding of the nucleic acid binding portion to a polynucleotide having or comprising the nucleotide sequence of the site; and/or
(6) testing the affinity and/or specificity of the binding of the molecule to a polynucleotide having or comprising the nucleotide sequence of the site; and/or
(7) testing the efficacy of the molecule or polynucleotide in modulating or suppressing the expression of the gene and/or of a reporter gene comprising the nucleotide sequence of the site.

40. An in-vitro method for suppressing the expression of a selected gene in a cell the method comprising introducing into the cell a molecule according to any of Claims 1 or 3 to 19.

41. An in-vitro method for modulating the expression of a selected gene in a cell the method comprising introducing into the cell a molecule according to any of Claims 2 to 19.

42. A method according to any of claims 34 to 41, wherein the cell is an eukaryotic cell.

43. A method according to any of claims 34 to 41 wherein the cell is a plant cell and is contained within a plant.

44. A method according to any of claims 34, 36, 38, 39, 40, 42 or 43, wherein the expression of a plurality of selected genes is suppressed.

## Patentansprüche

1. Molekül zur Unterdrückung der Expression eines ausgewählten Gens in einer Zelle, wobei das Molekül umfasst (1) einen Nucleinsäure-bindenden Abschnitt, der an den Ort des ausgewählten Gens oder an eine mit diesem assoziierte Stelle, wobei diese Stelle in einem Genom vorliegt, bindet, und (2) einen Abschnitt aus einem Expressionsrepressor, wobei der Nucleinsäure-bindende Abschnitt ein Oligonucleotid oder eine ein Oligonucleotid nachahmende Struktur oder ein Oligonucleotidanaloges umfasst, und wobei der Repressorabschnitt ein Polypeptid oder Peptidomimetikum umfasst.

2. Molekül zur Modulation der Expression eines ausgewählten Gens in einer Zelle, wobei das Molekül umfasst (1) einen Nucleinsäure-bindenden Abschnitt, der an den Ort des ausgewählten Gens oder an eine mit diesem assoziierte Stelle, wobei diese Stelle in einem Genom vorliegt, bindet, und (2) einen modifizierenden Abschnitt, wobei der Nucleinsäure-bindende Abschnitt ein Oligonucleotid oder eine ein Oligonucleotid nachahmende Struktur oder ein Oligonucleotidanaloges umfasst, und wobei der modifizierende Abschnitt ein Polypeptid oder Peptidomimetikum umfasst, das in der Lage ist, die kovalente Modifizierung der Nucleinsäure oder des Chromatins zu modulieren, und keine Endonuclease oder KRAB ist.

3. Molekül nach Anspruch 1 oder 2, wobei der Repressorabschnitt oder der modifizierende Abschnitt ein das Chromatin modifizierender Abschnitt und nicht KRAB ist.

4. Molekül nach Anspruch 1 oder 2, wobei der Repressorabschnitt oder der modifizierende Abschnitt aus einer vollständigen Komponente eines DNA-Methylase-Komplexes oder aus einem Teil von ihr besteht oder aus einem vollständigen Polypeptid, das an einen DNA-Methylase-Komplexes bindet oder dessen Rekrutierung fördert und das nicht KRAB ist, oder einem Teil von ihm besteht.

5. Molekül nach Anspruch 1 oder 2, wobei der Repressorabschnitt oder der modifizierende Abschnitt aus einer vollständigen Komponente einer Histonacetyltransferase oder aus einem Teil von ihr besteht oder aus einem vollständigen Polypeptid, das an einen Histonacetyltransferase-Komplex bindet oder dessen Rekrutierung fördert und das nicht KRAB ist, oder einem Teil von ihm besteht.

6. Molekül gemäß einem beliebigen der vorhergehenden Ansprüche, wobei der Polypeptid-oder Peptidomimetikumabschnitt des Moleküls eine Molekülmasse von unter 11 kDa hat.

7. Molekül gemäß einem beliebigen der vorhergehenden Ansprüche, wobei der Nucleinsäure-bindende Abschnitt ein DNA-bindender Abschnitt ist.

8. Molekül gemäß einem beliebigen der Ansprüche 1 bis 6, wobei der Nucleinsäure-bindende Abschnitt ein RNA-bindender Abschnitt ist und die in einem Genom vorliegende Stelle eine naszierende RNA ist, die von der DNA transkribiert wird.

9. Molekül nach einem beliebigen der vorhergehenden Ansprüche, wobei das Oligonucleotid oder Oligonucleotidanaloge oder die ein Oligonucleotid nachahmende Struktur ein ein Tripel bildendes Oligonucleotid (triple forming oligonucleotide, TFO) ist.

10. Molekül nach einem beliebigen der vorhergehenden Ansprüche, wobei das Oligonucleotidanaloge oder die ein Oligonucleotid, nachahmende Struktur eine Peptidnucleinsäure (peptide nucleic acid, PNA) ist.

11. Molekül gemäß Anspruch 3 oder von diesem abhängigen Ansprüchen, wobei der Chromatin-inaktivierende Abschnitt die Histondeacetylierung fördert.

12. Molekül gemäß Anspruch 3 oder von diesem abhängigen Ansprüchen oder 11, wobei der Chromatin-inaktivierende Abschnitt aus einer vollständigen Komponente eines Histondeacetylierung (HDAC)-Komplexes oder aus einem Teil von ihr besteht oder aus einem vollständigen Polypeptid, das an einen HDAC-Komplex bindet oder dessen Rekrutierung fördert, oder aus einem Teil von ihm besteht.

13. Molekül gemäß Anspruch 12, wobei die Komponente des HDAC-Komplexes oder das Polypeptid, das an einen HDAC-Komplex bindet oder dessen Rekrutierung fördert, eine beliebige bzw. ein beliebiges von PLZF, N-CoR, SMRT, Sin3, SAP18, SAP30, HDAC, NuRD, MAD1, MAD2, MAD3, MAD4, Rb oder E7 ist.

14. Molekül gemäß Anspruch 13, wobei der Chromatin-inaktivierende Abschnitt aus dem vollständigen PLZF oder aus einem N-CoR- oder SMRT-bindenden Abschnitt von ihm besteht.

15. Molekül gemäß Anspruch 13, wobei der Chromatin-inaktivierende Abschnitt aus einer vollständigen HDAC oder aus einem enzymatisch aktiven Teil von ihr besteht.

16. Molekül gemäß Anspruch 13, wobei der Chromatin-inaktivierende Abschnitt aus dem vollständigen E7 oder aus einem Histondeacetylase-Komplex-bindenden Teil von ihm besteht.

17. Molekül gemäß einem beliebigen der vorhergehenden Ansprüche, wobei das Molekül ferner einen Abschnitt umfasst, der den Eintritt in die Zelle und/oder die Lokalisation im Zellkern fördert.

18. Molekül gemäß Anspruch 17, wobei der Abschnitt, der den Eintritt in die Zelle und/oder die Lokalisation im Zellkern fördert, ein kleines Peptid aus 7-16 Aminosäuren ist, zum Beispiel die modifizierte Antennapedia-Homöodomäne gemäß der Sequenz RQIKIWFQNRRMKWKK oder das basische HIV-TAT-Internalisierungspeptid gemäß der Sequenz C(Acm)GRKKRRQRRRPQC, wobei C(Acm) Cys-Acetamidomethyl ist.

19. Molekül gemäß einem beliebigen der Ansprüche 1 bis 18, wobei der Nucleinsäure-bindende Abschnitt und der Repressorabschnitt oder modifizierende Abschnitt fusioniert sind.

20. Verwendung eines Moleküls gemäß einem beliebigen der Ansprüche 1 bis 19 bei der Herstellung eines Mittels zur Modulation der Expression des ausgewählten Gens in einer Zelle.

21. Verwendung nach Anspruch 20, wobei das Mittel zur Unterdrückung der Expression des ausgewählten Gens dient.

22. Verwendung gemäß Anspruch 20 oder 21, wobei das Mittel ein Medikament zur Modulierung oder Unterdrückung der Expression eines ausgewählten Gens in einem Tier ist, wobei das Tier Krebs hat.

23. Verwendung eines Moleküls gemäß einem beliebigen der Ansprüche 1 bis 19 bei der Herstellung eines Medikament zur Unterdrückung der Expression eines ausgewählten Gens in einem Patienten, der Krebs hat.

24. Molekül gemäß einem beliebigen der Ansprüche 1 bis 19 für die Verwendung in der Medizin.

25. Pharmazeutische Zusammensetzung, die ein Molekül gemäß einem beliebigen der Ansprüche 1 bis 19 und einen pharmazeutisch annehmbaren Träger umfasst.

26. Zusammensetzung nach Anspruch 25, die Mittel zur Förderung der Aufnahme des Moleküls in die Zelle umfasst.

27. Zusammensetzung nach Anspruch 26, wobei das Mittel zur Förderung der Aufnahme des Moleküls in die Zelle aus Liposomen oder einem viralen Träger besteht.

28. Wirtszelle, die ein Molekül gemäß einem beliebigen der Ansprüche 1 bis 19 aufweist.

29. Wirtszelle gemäß Anspruch 28, die eine Bakterienzelle ist.

30. Wirtszelle gemäß Anspruch 28, die eine Tierzelle ist.

31. Wirtszelle gemäß Anspruch 28, die eine Pflanzenzelle ist.

32. Tier, bei dem es sich nicht um einen Menschen handelt, das eine Wirtszelle gemäß Anspruch 30 aufweist.

33. Pflanze, die eine Wirtszelle gemäß Anspruch 31 aufweist.

34. Verfahren zur Konstruktion eines Moleküls zur Unterdrückung der Expression eines ausgewählten Gens in einer Zelle, wobei das Verfahren umfasst
(1) Identifizieren einer am Ort des ausgewählten Gens lokalisierten oder mit diesem assoziierten Stelle,
(2) Identifizieren oder Konstruieren eines Nucleinsäure-bindenden Abschnitts, der an die Stelle bindet, oder für den vorhergesagt wird, dass er an die Stelle bindet,
(3) Herstellen eines Moleküls, das den Nucleinsäure-bindenden Abschnitt und einen Abschnitt aus einem Expressionsrepressor umfasst,
wobei der Nucleinsäure-bindende Abschnitt ein Oligonucleotid oder eine ein Oligonucleotid nachahmende Struktur oder ein Oligonucleotidanaloges umfasst, und wobei der Repressorabschnitt ein Polypeptid oder Peptidomimetikum umfasst.

35. Verfahren zur Konstruktion eines Moleküls zur Modulation der Expression eines ausgewählten Gens in einer Zelle, wobei das Verfahren umfasst
(1) Identifizieren einer am Ort des ausgewählten Gens lokalisierten oder mit diesem assoziierten Stelle,
(2) Identifizieren oder Konstruieren eines Nucleinsäure-bindenden Abschnitts, der an die Stelle bindet, oder für den vorhergesagt wird, dass er an die Stelle bindet,
(3) Herstellen eines Moleküls, das den Nucleinsäure-bindenden Abschnitt und einen modifizierenden Abschnitt umfasst,
wobei der Nucleinsäure-bindende Abschnitt ein Oligonucleotid oder eine ein Oligonucleotid nachahmende Struktur oder ein Oligonucleotidanaloges umfasst, und wobei der modifizierende Abschnitt ein Polypeptid oder Peptidomimetikum umfasst, das in der Lage ist, die kovalente Modifizierung der Nucleinsäure oder des Chromatins zu modulieren.

36. Verfahren zur Konstruktion eines Moleküls zur Unterdrückung der Expression eines ausgewählten Gens in einer Zelle, wobei das Verfahren umfasst
(1) Identifizieren einer am Ort des ausgewählten Gens lokalisierten oder mit diesem assoziierten Stelle,
(2) Identifizieren oder Konstruieren eines Nucleinsäure-bindenden Abschnitts, der an ein Polynucleotid bindet, oder für den vorhergesagt wird, dass er an ein Polynucleotid bindet, das die Nucleotidsequenz der Stelle hat oder umfasst,
(3) Herstellen eines Moleküls, das den Nucleinsäure-bindenden Abschnitt und einen Abschnitt aus einem Expressionsrepressor umfasst,
wobei der Nucleinsäure-bindende Abschnitt ein Oligonucleotid oder eine ein Oligonucleotid nachahmende Struktur oder ein Oligonucleotidanaloges umfasst, und wobei der Repressorabschnitt ein Polypeptid oder Peptidomimetikum umfasst.

37. Verfahren zur Konstruktion eines Moleküls zur Modulation der Expression eines ausgewählten Gens in einer Zelle, wobei das Verfahren umfasst
(1) Identifizieren einer am Ort des ausgewählten Gens lokalisierten oder mit diesem assoziierten Stelle,
(2) Identifizieren oder Konstruieren eines Nucleinsäure-bindenden Abschnitts, der an ein Polynucleotid bindet, oder für den vorhergesagt wird, dass er an ein Polynucleotid bindet, das die Nucleotidsequenz der Stelle hat oder umfasst,
(3) Herstellen eines Moleküls, das den Nucleinsäure-bindenden Abschnitt und einen modifizierenden Abschnitt umfasst,
wobei der Nucleinsäure-bindende Abschnitt ein Oligonucleotid oder eine ein Oligonucleotid nachahmende Struktur oder ein Oligonucleotidanaloges umfasst, und wobei der modifizierende Abschnitt ein Polypeptid oder Peptidomimetikum umfasst, das in der Lage ist, die kovalente Modifizierung der Nucleinsäure oder des Chromatins zu modulieren.

38. Verfahren nach Anspruch 34 oder 35, das ferner die folgenden Schritte umfasst:
(4) Durchführen einer Kontrolle der Qualität der Molekülpräparation um festzustellen, dass der Nucleinsäure-bindende Abschnitt und der Repressorabschnitt oder modifizierende Abschnitt aneinander befestigt sind; und/oder
(5) Testen der Affinität und/oder Spezifität der Bindung des Nucleinsäure-bindenden Abschnitts an die Stelle; und/oder
(6) Testen der Affinität und/oder Spezifität der Bindung des Moleküls an die Stelle; und/oder
(7) Testen der Wirksamkeit des Moleküls oder Polynucleotids bezüglich der Modulierung oder Unterdrückung der Expression des Gens und/oder eines Reportergens, das die Nucleotidsequenz der Stelle umfasst.

39. Verfahren nach Anspruch 36 oder 37, das ferner die folgenden Schritte umfasst:
(4) Durchführen einer Kontrolle der Qualität der Molekülpräparation um zu bestimmen, dass der Nucleinsäure-bindende Abschnitt und der Repressorabschnitt oder der modifizierende Abschnitt aneinander befestigt sind; und/oder
(5) Testen der Affinität und/oder Spezifität der Bindung des Nucleinsäure-bindenden Abschnitts an ein Polynucleotid, das die Nucleotidsequenz der Stelle hat oder umfasst; und/oder
(6) Testen der Affinität und/oder Spezifität der Bindung des Moleküls an ein Polynucleotid, das die Nucleotidsequenz der Stelle hat oder umfasst; und/oder
(7) Testen der Wirksamkeit des Moleküls oder Polynucleotids bezüglich der Modulierung oder Unterdrückung der Expression des Gens und/oder eines Reportergens, das die Nucleotidsequenz der Stelle umfasst.

40. In-vitro-Verfahren zur Unterdrückung der Expression eines ausgewählten Gens in einer Zelle, wobei das Verfahren das Einbringen eines Moleküls gemäß einem beliebigen der Ansprüche 1 oder 3 bis 19 in die Zelle umfasst.

41. In-vitro-Verfahren zur Modulation der Expression eines ausgewählten Gens in einer Zelle, wobei das Verfahren das Einbringen eines Moleküls gemäß einem beliebigen der Ansprüche 2 bis 19 in die Zelle umfasst.

42. Verfahren gemäß einem beliebigen der Ansprüche 34 bis 41, wobei die Zelle eine eukaryotische Zelle ist.

43. Verfahren gemäß einem beliebigen der Ansprüche 34 bis 41, wobei die Zelle eine Pflanzenzelle ist und in einer Pflanze enthalten ist.

44. Verfahren gemäß einem beliebigen der Ansprüche 34, 36, 38, 39, 40, 42 oder 43, wobei die Expression einer Vielzahl ausgewählter Gene unterdrückt wird.

## Revendications

1. Molécule pour la suppression de l'expression d'un gène choisi dans une cellule comprenant (1) une portion de liaison d'acide nucléique qui se lie à un site au niveau d'un ou associé à un gène choisi dont le site est présent dans un génome et (2) une portion de répression de l'expression, dans laquelle la portion de liaison d'acide nucléique comprend un oligonucléotide ou un mimétique oligonucléotidique ou un analogue oligonucléotidique, et dans laquelle la portion de répression comprend un polypeptide ou un peptidomimétique.

2. Molécule pour la modulation de l'expression d'un gène choisi dans une cellule comprenant (1) une portion de liaison d'acide nucléique qui se lie à un site au niveau d'un ou associé à un gène choisi dont le site est présent dans un génome et (2) une portion de modification, dans laquelle la portion de liaison d'acide nucléique comprend un oligonucléotide ou un mimétique oligonucléotidique ou un analogue oligonucléotidique, et dans laquelle la portion de modification comprend un polypeptide ou un peptidomimétique qui est capable de moduler une modification covalente d'acide nucléique ou de chromatine et n'est pas une endonucléase ou KRAB.

3. Molécule selon la revendication 1 ou 2, dans laquelle la portion de répression ou de modification est une portion d'inactivation de chromatine et n'est pas KRAB.

4. Molécule selon la revendication 1 ou 2, dans laquelle la portion de répression ou de modification est l'intégralité ou une portion d'un composant d'un complexe d'ADN méthylase ou l'intégralité ou une portion d'un polypeptide qui se lie au ou facilite le recrutement d'un complexe d'ADN méthylase et n'est pas KRAB.

5. Molécule selon la revendication 1 ou 2, dans laquelle la portion de répression ou de modification est l'intégralité ou une portion d'un composant d'une histone acétyltransférase ou l'intégralité ou une portion d'un polypeptide qui se lie au ou facilite le recrutement d'un complexe d'histone acétyltransférase et n'est pas KRAB.

6. Molécule selon l'une quelconque des revendications précédentes, dans laquelle le polypeptide ou la partie peptidomimétique de la molécule a une masse moléculaire inférieure à 11 kDa.

7. Molécule selon l'une quelconque des revendications précédentes, dans laquelle la portion de liaison d'acide nucléique est une portion de liaison d'ADN.

8. Molécule selon l'une quelconque des revendications 1 à 6, dans laquelle la portion de liaison d'acide nucléique est une portion de liaison d'ARN et le site présent dans un génome est un ARN naissant en cours de transcription à partir de l'ADN.

9. Molécule selon l'une quelconque des revendications précédentes, dans laquelle l'oligonucléotide ou l'analogue oligonucléotidique ou le mimétique oligonucléotidique est un oligonucléotide formant une triple hélice (TFO).

10. Molécule selon l'une quelconque des revendications précédentes, dans laquelle l'analogue oligonucléotidique ou le mimétique oligonucléotidique est un acide nucléique peptidique (PNA).

11. Molécule selon la revendication 3 ou les revendications dépendantes de celle-ci, dans laquelle la portion d'inactivation de chromatine facilite la déacétylation des histones.

12. Molécule selon la revendication 3 ou les revendications dépendantes de celle-ci ou la revendication 11, dans laquelle la portion d'inactivation de chromatine est l'intégralité ou une portion d'un composant d'un complexe de déacétylation des histones (HDAC), ou l'intégralité ou une portion d'un polypeptide qui se lie au ou facilite le recrutement d'un complexe HDAC.

13. Molécule selon la revendication 12, dans laquelle le composant du complexe HDAC ou le polypeptide qui se lie au ou facilite le recrutement d'un complexe HDAC est l'un quelconque parmi PLZF, N-CoR, SMRT, Sin3, SAP18, SAP30, HDAC, NuRD, MAD1, MAD2, MAD3, MAD4, Rb ou E7.

14. Molécule selon la revendication 13, dans laquelle la portion d'inactivation de chromatine est l'intégralité ou une partie de liaison à N-CoR ou à SMRT de PLZF.

15. Molécule selon la revendication 13, dans laquelle la portion d'inactivation de chromatine est l'intégralité ou une partie enzymatiquement active d'un HDAC.

16. Molécule selon la revendication 13, dans laquelle la portion d'inactivation de chromatine est l'intégralité ou une partie de liaison au complexe d'histone déacétylase de E7.

17. Molécule selon l'une quelconque des revendications précédentes, dans laquelle la molécule comprend en outre une portion qui facilite l'entrée cellulaire et/ou la localisation nucléaire.

18. Molécule selon la revendication 17, dans laquelle la portion qui facilite l'entrée cellulaire et/ou la localisation nucléaire est un petit peptide de 7 à 16 acides aminés, par exemple un homéodomaine *d'Antennapedia* modifié selon la séquence RQIKIWFQNRRMKWKK ou un peptide d'intériorisation TAT du VIH basique selon la séquence C (Acm) GRKKRRQRRRPQC, où C(Acm) est une Cys-acétamidométhyle.

19. Molécule selon l'une quelconque des revendications 1 à 18, dans laquelle la portion de liaison d'acide nucléique et la portion de répression ou de modification sont fusionnées.

20. Utilisation d'une molécule selon l'une quelconque des revendications 1 à 19, pour la préparation d'un agent destiné à moduler l'expression du gène choisi dans une cellule.

21. Utilisation selon la revendication 20, dans laquelle l'agent est destiné à supprimer l'expression du gène choisi.

22. Utilisation selon la revendication 20 ou 21,
dans laquelle l'agent est un médicament destiné à moduler ou à supprimer l'expression d'un gène choisi chez un animal, dans laquelle l'animal a un cancer.

23. Utilisation d'une molécule selon l'une quelconque des revendication 1 à 19, pour la préparation d'un médicament destiné à supprimer l'expression d'un gène choisi chez un patient qui a un cancer.

24. Molécule selon l'une quelconque des revendications 1 à 19, pour l'utilisation en médecine.

25. Composition pharmaceutique comprenant une molécule selon l'une quelconque des revendications 1 à 19 et un véhicule pharmaceutiquement acceptable.

26. Composition selon la revendication 25, comprenant des moyens pour favoriser la capture cellulaire de la molécule.

27. Composition selon la revendication 26, dans laquelle les moyens pour favoriser la capture cellulaire de la molécule sont des liposomes ou un véhicule viral.

28. Cellule hôte comprenant une molécule selon l'une quelconque des revendications 1 à 19.

29. Cellule hôte selon la revendication 28 qui est une cellule bactérienne.

30. Cellule hôte selon la revendication 28 qui est une cellule animale.

31. Cellule hôte selon la revendication 28 qui est une cellule végétale.

32. Animal non humain comprenant une cellule hôte selon la revendication 30.

33. Plante comprenant une cellule hôte selon la revendication 31.

34. Méthode pour mettre au point une molécule pour supprimer l'expression d'un gène choisi dans une cellule, la méthode comprenant les étapes consistant à :
(1) identifier un site au niveau du ou associé au gène choisi ;
(2) identifier ou mettre au point une portion de liaison d'acide nucléique qui se lie ou est supposée se lier au site ;
(3) préparer une molécule comprenant la portion de liaison d'acide nucléique et une portion de répression d'expression ;
dans laquelle la portion de liaison d'acide nucléique comprend un oligonucléotide ou un mimétique oligonucléotidique ou un analogue oligonucléotidique, et dans laquelle la portion de répression comprend un polypeptide ou un peptidomimétique.

35. Méthode pour mettre au point une molécule pour moduler l'expression d'un gène choisi dans une cellule, la méthode comprenant les étapes consistant à :
(1) identifier un site au niveau du ou associé au gène choisi ;
(2) identifier ou mettre au point une portion de liaison d'acide nucléique qui se lie ou est supposée se lier au site ;
(3) préparer une molécule comprenant la portion de liaison d'acide nucléique et une portion de modification ;
dans laquelle la portion de liaison d'acide nucléique comprend un oligonucléotide ou un mimétique oligonucléotidique ou un analogue oligonucléotidique, et dans laquelle la portion de modification comprend un polypeptide ou un peptidomimétique qui est capable de moduler la modification covalente d'acide nucléique ou de chromatine.

36. Méthode pour mettre au point une molécule pour supprimer l'expression d'un gène choisi dans une cellule, la méthode comprenant les étapes consistant à :
(1) identifier un site au niveau du ou associé au gène choisi ;
(2) identifier ou mettre au point une portion de liaison d'acide nucléique qui se lie ou est supposée se lier à un polynucléotide ayant ou comprenant la séquence nucléotidique du site ;
(3) préparer une molécule comprenant la portion de liaison d'acide nucléique et une portion de répression de l'expression ;
dans laquelle la portion de liaison d'acide nucléique comprend un oligonucléotide ou un mimétique oligonucléotidique ou un analogue oligonucléotidique, et dans laquelle la portion de répression comprend un polypeptide ou un peptidomimétique.

37. Méthode pour mettre au point une molécule pour moduler l'expression d'un gène choisi dans une cellule, la méthode comprenant les étapes consistant à :
(1) identifier un site au niveau du ou associé au gène choisi ;
(2) identifier ou mettre au point une portion de liaison d'acide nucléique qui se lie ou est supposée se lier à un polynucléotide ayant ou comprenant la séquence nucléotidique du site ;
(3) préparer une molécule comprenant la portion de liaison d'acide nucléique et une portion de modification ;
dans laquelle la portion de liaison d'acide nucléique comprend un oligonucléotide ou un mimétique oligonucléotidique ou un analogue oligonucléotidique, et dans laquelle la portion de modification comprend un polypeptide ou un peptidomimétique qui est capable de moduler la modification covalente d'acide nucléique ou de chromatine.

38. Méthode selon la revendication 34 ou 35, comprenant en outre les étapes consistant à :
(4) réaliser une évaluation de contrôle qualité sur la préparation de la molécule dans le but d'établir que la portion de liaison d'acide nucléique et la portion de répression ou de modification sont liées l'une à l'autre ; et/ou
(5) tester l'affinité et/ou la spécificité de liaison de la portion de liaison d'acide nucléique au site ; et/ou
(6) tester l'affinité et/ou la spécificité de liaison de la molécule au site ; et/ou
(7) tester l'efficacité de la molécule ou du polynucléotide pour moduler ou supprimer l'expression du gène et/ou d'un gène rapporteur comprenant la séquence nucléotidique du site.

39. Méthode selon la revendication 36 ou 37, comprenant en outre les étapes consistant à :
(4) réaliser une évaluation de contrôle qualité sur la préparation de la molécule dans le but d'établir que la portion de liaison d'acide nucléique et la portion de répression ou de modification sont liées l'une à l'autre ; et/ou
(5) tester l'affinité et/ou la spécificité de liaison de la portion de liaison d'acide nucléique à un polynucléotide ayant ou comprenant la séquence nucléotidique du site ; et/ou
(6) tester l'affinité et/ou la spécificité de la liaison de la molécule à un polynucléotide ayant ou comprenant la séquence nucléotidique du site ; et/ou
(7) tester l'efficacité de la molécule ou du polynucléotide pour moduler ou supprimer l'expression du gène et/ou d'un gène rapporteur comprenant la séquence nucléotidique du site.

40. Méthode *in vitro* de suppression de l'expression d'un gène choisi dans une cellule, la méthode comprenant l'étape consistant à introduire dans la cellule une molécule selon l'une quelconque des revendications 1 ou 3 à 19.

41. Méthode *in vitro* de modulation de l'expression d'un gène choisi dans une cellule, la méthode comprenant l'étape consistant à introduire dans la cellule une molécule selon l'une quelconque des revendications 2 à 19.

42. Méthode selon l'une quelconque des revendications 34 à 41, dans laquelle la cellule est une cellule eucaryote.

43. Méthode selon l'une quelconque des revendications 34 à 41, dans laquelle la cellule est une cellule végétale et est contenue au sein d'une plante.

44. Méthode selon l'une quelconque des revendications 34, 36, 38, 39, 40, 42 ou 43, dans laquelle l'expression d'une pluralité de gènes choisis est supprimée.
